# EUROPEAN PATENT APPLICATION

(11) **EP 2 860 264 A1**
(43) Date of publication of application: **15.04.2015**
(21) Application number: 14188085.6
(22) Date of filing: 08.10.2014
(51) Int. Cl.: C12Q 1/68

(54) **Nucleic acid amplification method, nucleic acid extraction device, nucleic acid amplification reaction cartridge, and nucleic acid amplification reaction kit**

(30) Priority: 09.10.2013 JP 2013212253
(71) Applicant: Seiko Epson Corporation, Tokyo 163 (JP)
(72) Inventor: Yamada, Kiyohito, Nagano, 392-8502 (JP); Hanamura, Masato, Nagano, 392-8502 (JP); Saito, Yuji, Nagano, 392-8502 (JP)
(74) Representative: Hoffmann Eitle

(57) **Abstract**

A nucleic acid amplification method includes: adsorbing a nucleic acid to fine particles by mixing a chaotropic substance-containing adsorbent liquid and the fine particles with a nucleic acid-containing sample; washing the fine particles adsorbing the nucleic acid with a first washing liquid; eluting the nucleic acid adsorbed to the fine particles in an eluate; and performing a nucleic acid amplification reaction on the nucleic acid in the eluate, the adsorbent liquid contains an alcohol, and the first washing liquid is acidic. This method can be carried out by a nucleic acid extraction device, a nucleic acid amplification reaction cartridge, and a nucleic acid amplification reaction kit.

## Description

### BACKGROUND

### 1. Technical Field

The present invention relates to a nucleic acid amplification method, a nucleic acid extraction device, a nucleic acid amplification reaction cartridge, and a nucleic acid amplification reaction kit.

### 2. Related Art

In recent years, medical care using genes, such as gene diagnosis and gene therapy, has attracted attention due to the development of techniques for using genes. In the agriculture and stockbreeding field, many methods using genes have also been developed for variety determination and breeding. A technique such as polymerase chain reaction (PCR) is widely used as a technique for using genes. Nowadays, the PCR is an essential technique in analyzing the information of a biological substance. The PCR is a method of applying a thermal cycle to a solution (reaction liquid) containing a nucleic acid (target nucleic acid) which is an amplification object and a reagent to amplify the target nucleic acid. As for the thermal cycle of the PCR, a method of applying a thermal cycle at two-or three-stage temperatures is generally used.

Currently, a simple examination kit such as an immunochromatography kit is mainly used for diagnosis of infections represented by influenza in the medical practice. However, in such a simple examination, the accuracy thereof may be insufficient, and PCR with which higher examination accuracy can be expected is desired to be applied to the diagnosis of infections. In general outpatient practices and the like in medical institutions, the time which can be spent for examination is limited to a short time since the time for diagnosis is limited. Therefore, for example, identification of influenza has been performed at the sacrifice of examination accuracy to reduce the time with simple examination such as immunochromatography.

Due to such a circumstance, it is necessary to reduce the time required for reaction in order to realize the examination by PCR with which higher accuracy can be expected. For example, JP-A-2009-136250 discloses, as an apparatus for causing a PCR in a short time, a biological sample reactor in which a chip for a biological sample reaction filled with a reaction liquid and a liquid which does not mix with the reaction liquid and has a lower specific gravity than the reaction liquid is rotated around a horizontal rotary shaft to move the reaction liquid, thereby applying a thermal cycle (JP-A-2009-136250). In addition, a method using magnetic beads (JP-A-2009-207459), a method using magnetic beads as a droplet moving section to move droplets in a temperature changeable area on a substrate, thereby applying a thermal cycle of PCR (JP-A-2008-012490), and the like are disclosed as one method for PCR.

### SUMMARY

An advantage of some aspects of the invention is that it provides a nucleic acid amplification method, a nucleic acid extraction device, a nucleic acid amplification reaction cartridge, and a nucleic acid amplification reaction kit.

A nucleic acid amplification method according to an aspect of the invention includes: adsorbing a nucleic acid to fine particles by mixing a chaotropic substance-containing adsorbent liquid and the fine particles with a nucleic acid-containing sample; washing the fine particles adsorbing the nucleic acid with a first washing liquid; eluting the nucleic acid adsorbed to the fine particles in an eluate; and performing a nucleic acid amplification reaction on the nucleic acid in the eluate, the adsorbent liquid contains an alcohol, and the first washing liquid is acidic.

A nucleic acid amplification method according to another aspect of the invention includes: adsorbing a nucleic acid to fine particles by mixing a chaotropic substance-containing adsorbent liquid and the fine particles with a nucleic acid-containing sample; washing the fine particles adsorbing the nucleic acid with a second washing liquid; washing, with a first washing liquid, the fine particles adsorbing the nucleic acid after the washing with the second washing liquid; eluting the nucleic acid adsorbed to the fine particles in an eluate; and amplifying the nucleic acid, the second washing liquid contains an alcohol or acetonitrile, and the first washing liquid is acidic.

In the nucleic acid amplification method according to any one of the aspects of the invention described above, the alcohol may be methanol or ethanol. The adsorbent liquid may have an alcohol concentration of 40% to 50% by volume. The nucleic acid may be a ribonucleic acid (RNA), reverse-transcribing the ribonucleic acid eluted in the eluate to synthesize cDNA may be further included, and the nucleic acid which is amplified in the amplifying the nucleic acid may be the synthesized cDNA. The reaction liquid in the amplifying the nucleic acid may contain 50% by volume or more of the eluate. The reaction liquid in the amplifying the nucleic acid may be the eluate.

A nucleic acid extraction device according to still another aspect of the invention includes: a tube having a longitudinal direction in which a first plug formed of an oil, a second plug formed of a first washing liquid which is phase-separated from an oil and is provided to wash fine particles to which a nucleic acid is bonded, a third plug formed of an oil, a fourth plug formed of an eluate which is phase-separated from an oil and is provided to elute the nucleic acid from the fine particles to which the nucleic acid is bonded, and a fifth plug formed of an oil are provided in order; and a container which is capable of being connected to and allowed to communicate with a side of the tube where the first plug is disposed, and which contains an adsorbent liquid for adsorbing the nucleic acid to the fine particles, the adsorbent liquid contains an alcohol, and the first washing liquid is acidic.

A nucleic acid extraction device according to yet another aspect of the invention includes: a tube having a longitudinal direction in which a first plug formed of an oil, a second plug formed of a first washing liquid which is phase-separated from an oil and is provided to wash fine particles to which a nucleic acid is bonded, a third plug formed of an oil, a fourth plug formed of an eluate which is phase-separated from an oil and is provided to elute the nucleic acid from the fine particles to which the nucleic acid is bonded, and a fifth plug formed of an oil are provided in order; and a liquid reservoir which communicates with a side of the tube where the first plug is disposed, and which contains an adsorbent liquid for adsorbing the nucleic acid to the fine particles, the adsorbent liquid contains an alcohol, and the first washing liquid is acidic.

In the nucleic acid extraction device according to any one of the aspects of the invention described above, the alcohol may be methanol or ethanol. The adsorbent liquid may have an alcohol concentration of 40% to 50% by volume.

A nucleic acid extraction device according to still another aspect of the invention includes: a tube having a longitudinal direction in which a first plug formed of an oil, a sixth plug formed of a second washing liquid which is phase-separated from an oil and is provided to wash fine particles to which a nucleic acid is bonded, a seventh plug formed of an oil, a second plug formed of a first washing liquid which is phase-separated from an oil and is provided to wash the fine particles, to which the nucleic acid is bonded, washed with the second washing liquid, a third plug formed of an oil, a fourth plug formed of an eluate which is phase-separated from an oil and is provided to elute the nucleic acid from the fine particles to which the nucleic acid is bonded, and a fifth plug formed of an oil are provided in order, the second washing liquid contains an alcohol or acetonitrile, and the first washing liquid is acidic. Here, a container which is capable of being connected to and allowed to communicate with to a side of the tube where the first plug is disposed, and which contains an adsorbent liquid for adsorbing the nucleic acid to the fine particles may be provided, and the adsorbent liquid may contain an alcohol. Otherwise, a liquid reservoir which communicates with a side of the tube where the first plug is disposed, and which contains an adsorbent liquid for adsorbing the nucleic acid to the fine particles may be provided, and the adsorbent liquid may contain an alcohol. The alcohol contained in the second washing liquid may be methanol or ethanol. The concentration of the alcohol in the second washing liquid may be 70% to 100% by volume.

A nucleic acid amplification reaction cartridge according to further another aspect of the invention includes: the nucleic acid extraction device according to any one of the aspects of the invention described above; and a nucleic acid amplification reaction container which communicates with a side of the tube where the fifth plug is disposed, and which contains an oil. A reaction liquid of a nucleic acid amplification reaction may contain 50% by volume or more of an eluate in which the nucleic acid is eluted. The reaction liquid of the nucleic acid amplification reaction may be the eluate. A tank which communicates with a side of the tube where the first plug is disposed, and which introduces the fine particles to the tube may be provided.

A nucleic acid amplification reaction kit according to still further another aspect of the invention includes: the nucleic acid extraction device including a tube having a longitudinal direction in which a first plug formed of an oil, a sixth plug formed of a second washing liquid which is phase-separated from an oil and is provided to wash fine particles to which a nucleic acid is bonded, a seventh plug formed of an oil, a second plug formed of a first washing liquid which is phase-separated from an oil and is provided to wash the fine particles, to which the nucleic acid is bonded, washed with the second washing liquid, a third plug formed of an oil, a fourth plug formed of an eluate which is phase-separated from an oil and is provided to elute the nucleic acid from the fine particles to which the nucleic acid is bonded, and a fifth plug formed of an oil are provided in order, the second washing liquid containing an alcohol or acetonitrile, and the first washing liquid having an acidic pH; and a tank which introduces the fine particles to the tube.

### BRIEF DESCRIPTION OF THE DRAWINGS

The invention will be described with reference to the accompanying drawings, wherein like numbers reference like elements.
Fig. 1 is a diagram schematically illustrating a main part of a nucleic acid extraction device according to an embodiment.
Fig. 2 is a diagram schematically illustrating a main part of a nucleic acid extraction device according to an embodiment.
Fig. 3 is a diagram schematically illustrating a main part of a nucleic acid extraction device according to an embodiment.
Fig. 4A is a diagram schematically illustrating a nucleic acid extraction device according to an embodiment.
Fig. 4B is a diagram schematically illustrating a nucleic acid extraction device according to an embodiment.
Fig. 5 is a diagram schematically illustrating a main part of a nucleic acid extraction device according to an embodiment.
Figs. 6A and 6B are diagrams schematically illustrating a nucleic acid amplification reaction container of a nucleic acid extraction cartridge according to an embodiment.
Fig. 7A is a diagram schematically illustrating an example of a nucleic acid extraction kit according to an embodiment.
Fig. 7B is a diagram schematically illustrating an example of a nucleic acid extraction kit according to an embodiment.
Fig. 8 is a diagram schematically illustrating an example of a nucleic acid extraction cartridge according to an embodiment.
Fig. 9 is a schematic diagram for illustrating a modification example of the nucleic acid extraction method according to an embodiment.
Fig. 10 is a perspective view illustrating an example of a nucleic acid extraction apparatus according to an embodiment.
Fig. 11 is a perspective view illustrating an example of a nucleic acid extraction apparatus according to an embodiment.
Fig. 12 shows graphs illustrating results of Experimental Examples 1 and 2 using batching.
Fig. 13 shows graphs illustrating results of Experimental Examples 3 and 4 using a nucleic acid extraction device.
Fig. 14 is a graph illustrating a result of an example using a nucleic acid extraction device.

### DESCRIPTION OF EXEMPLARY EMBODIMENTS

Hereinafter, several embodiments of the invention will be described. Embodiments to be described below are provided to describe examples of the invention. The invention is not limited to the following embodiments, and includes various modifications implemented without changing the gist of the invention. The entire configuration to be described below is not necessarily an essential constituent element of the invention.

### 1. Nucleic Acid Extraction Device

A nucleic acid extraction device 1000 of this embodiment has a tube portion 100, a first plug 10, a second plug 20, a third plug 30, a fourth plug 40, and a fifth plug 50.

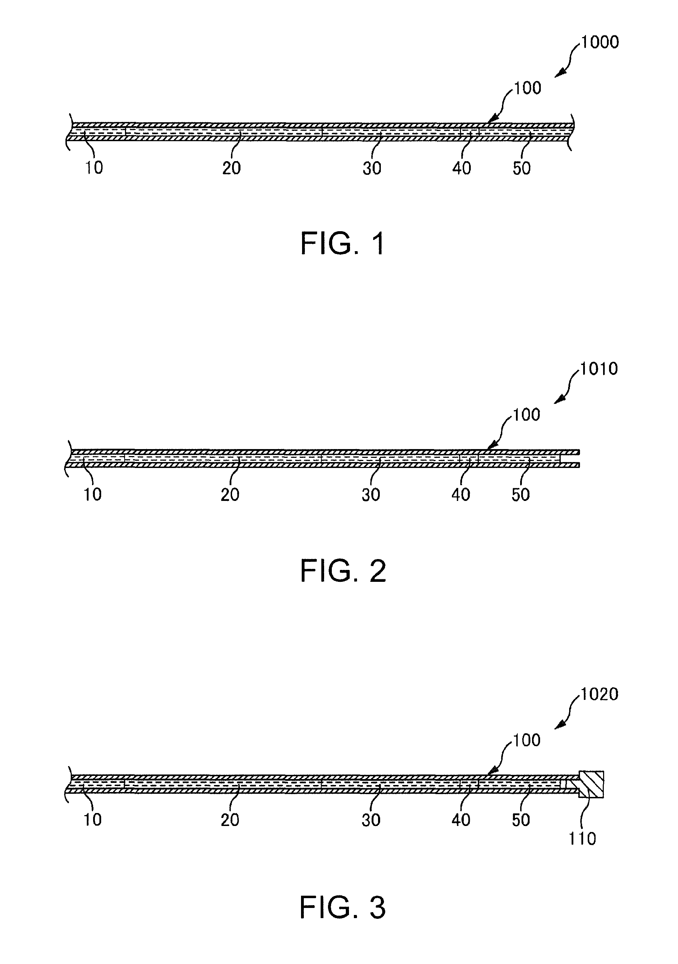

Fig. 1 is a diagram schematically illustrating a main part of the nucleic acid extraction device 1000 of this embodiment.

### 1.1. Tube Portion

The tube portion 100 constitutes a main part of the nucleic acid extraction device 1000. The nucleic acid extraction device 1000 may include various configurations other than the tube portion 100. Although not illustrated in Fig. 1, the nucleic acid extraction device 1000 may include a pipe, a container, a cock, a joint, a pump, a controller, and the like connected to the tube portion 100.

The tube portion 100 is a tubular part which has a cavity therein and can allow a liquid to flow in the cavity in a longitudinal direction. The tube portion 100 is in the longitudinal direction, but may be bent. The cavity in the tube portion 100 is not particularly limited in size and shape as long as the liquid accommodated therein can maintain a plug shape in the tube portion 100. The size of the internal cavity and the shape of a cross-section perpendicular to the longitudinal direction of the tube portion 100 may be changed in the longitudinal direction of the tube portion 100. Whether the liquid can maintain a plug shape in the tube portion 100 depends on conditions such as a material of the tube portion 100 and the kind of the liquid, and thus the shape of the cross-section perpendicular to the longitudinal direction of the tube portion 100 is appropriately designed within a range in which the liquid can maintain a plug shape in the tube portion 100.

The shape of the cross-section perpendicular to the longitudinal direction of the external form of the tube portion 100 is also not limited. The thickness (a length from a side surface of the internal cavity to an external surface) of the tube portion 100 is also not particularly limited. When the cross-section perpendicular to the longitudinal direction of the internal cavity of the tube portion 100 has an annular shape, the internal diameter (a diameter of the circle of the cross-section perpendicular to the longitudinal direction of the internal cavity) of the tube portion 100 can be set to, for example, 0.5 mm to 3 mm. It is preferable that the internal diameter of the tube portion 100 be within this range since a liquid plug is easily formed with a wide range of the material of the tube portion 100 and the kind of the liquid.

The material of the tube portion 100 is not particularly limited, but for example, glass, a polymer, a metal or the like can be used. It is preferable that a material having transparency with respect to visible light, such as glass and a polymer, be selected as the material of the tube portion 100 since the inside (the inside of the cavity) can be observed from the outside of the tube portion 100. It is preferable that a substance transmitting a magnetic force or a non-magnetic body be selected as the material of the tube portion 100 since when magnetic particles pass through the tube portion 100, it is easily performed by giving a magnetic force from the outside of the tube portion 100.

In the tube portion 100, the first plug 10 formed of a first oil, the second plug 20 formed of a first washing liquid which is phase-separated from an oil when mixing therewith and is provided to wash fine particles to which a nucleic acid is bonded, the third plug 30 formed of a second oil which does not mix with the first washing liquid, the fourth plug 40 formed of an eluate which is phase-separated from an oil when mixing therewith and is provided to elute the nucleic acid from the fine particles to which the nucleic acid is bonded, and the fifth plug 50 formed of a third oil which does not mix with the eluate are arranged in this order.

### 1.2. First Plug, Third Plug, and Fifth Plug

The first plug 10, the third plug 30, and the fifth plug 50 are all formed of an oil. The oils of the first plug 10, the third plug 30, and the fifth plug 50 may be different kinds of oils or the same kind of oil. As the oil, for example, one kind selected from silicone oils such as dimethylsilicone oils, paraffin oils, mineral oils, and mixtures thereof can be selected. The liquids which form the first plug 10, the second plug 20, the third plug 30, the fourth plug 40, and the fifth plug 50 next to each other are selected so as not to mix with each other.

The second plug 20 is disposed between the first plug 10 and the third plug 30. Another liquid plug may be disposed in an area on the side of the first plug 10 opposite the second plug 20. It is preferable that bubbles and other liquids do not exist in the first plug 10. However, as long as particles adsorbing a nucleic acid can pass through the first plug 10, bubbles and other liquids may exist. In addition, it is preferable that bubbles and other liquids do not exist between the first plug 10 and the second plug 20. However, as long as particles adsorbing a nucleic acid can pass through from the first plug 10 to the second plug 20, bubbles and other liquids may exist. Similarly, it is preferable that bubbles and other liquids do not exist between the second plug 20 and the third plug 30. However, as long as particles adsorbing a nucleic acid can pass through from the second plug 20 to the third plug 30, bubbles and other liquids may exist.

The fourth plug 40 is disposed between the third plug 30 and the fifth plug 50. Another liquid plug may be disposed in an area on the side of the fifth plug 50 opposite the fourth plug 40. It is preferable that bubbles and other liquids do not exist in the third plug 30. However, as long as particles adsorbing a nucleic acid can pass through the third plug 30, bubbles and other liquids may exist. In addition, it is preferable that bubbles and other liquids do not exist between the third plug 30 and the fourth plug 40. However, as long as particles adsorbing a nucleic acid can pass through from the third plug 30 to the fourth plug 40, bubbles and other liquids may exist. Similarly, it is preferable that bubbles and other liquids do not exist between the fourth plug 40 and the fifth plug 50. However, as long as particles adsorbing a nucleic acid can pass through from the fourth plug 40 to the fifth plug 50, bubbles and other liquids may exist. Furthermore, it is preferable that bubbles and other liquids do not exist in the fifth plug 50.

The lengths of the first plug 10, the third plug 30, and the fifth plug 50 in the longitudinal direction of the tube portion 100 are not particularly limited within a range in which the plugs can be formed. The lengths of the first plug 10, the third plug 30, and the fifth plug 50 in the longitudinal direction of the tube portion 100 are specifically 1 mm to 50 mm. In order to keep a moving distance of particles from increasing excessively, the lengths are preferably 1 mm to 30 mm, and more preferably 5 mm to 20 mm. Among these, when the length of the third plug 30 in the longitudinal direction of the tube portion 100 is increased, it is possible to make it harder for the second plug 20 to be discharged when an aspect in which the fourth plug 40 is discharged from an end on the side of the fifth plug 50 of the tube portion 100 is employed. In this case, a specific length of the third plug 30 can be set to 10 mm to 50 mm.

The first plug 10 and the fifth plug 50 function to prevent the first washing liquid (second plug 20) and the eluate (fourth plug 40) from being subjected to substance exchange with the outside air such as vaporization or from being contaminated from outside even when at least one end of the tube portion 100 is open. Therefore, even when at least one end of the tube portion 100 is open to the outside air, the volumes of the first washing liquid and the eluate can be kept constant, and thus a fluctuation in concentration of each liquid and contamination can be suppressed. Accordingly, it is possible to increase the accuracy of the concentrations of the nucleic acid and various agents in the nucleic acid extraction.

The third plug 30 functions to suppress the mixing of the first washing liquid (second plug 20) and the eluate (fourth plug 40). In addition, when the third plug 30 is formed of an oil having a higher viscosity, a "wiping effect" of the oil can be increased when particles are moved in an interface with the first washing liquid (second plug 20). Accordingly, when particles are moved from the first washing liquid plug which is the second plug 20 to the third oil plug 30, it is possible to make it harder for a water-soluble component adhering to the particles to be brought into the third plug 30 (oil).

### 1.3. Second Plug

The second plug 20 is disposed at a position between the first plug 10 and the third plug 30 in the tube portion 100. The second plug 20 is formed of a first washing liquid for washing fine particles to which a nucleic acid is bonded. The first washing liquid is a liquid which does not mix with both the oil of the first plug 10 and the oil of the third plug 30.

The first washing liquid is an acidic solution, and is particularly preferably an acidic aqueous solution. The acid to be contained is not particularly limited. However, an aqueous solution of a citric acid, an acetic acid, glycin hydrochloride, or the like is preferred. The first washing liquid may contain an ethylenediaminetetraacetic acid (EDTA) or a surfactant (Triton, Tween, SDS or the like), but is preferably a solution which does not substantially contain a chaotropic substance. The pH is not particularly limited as long as the first washing liquid is acidic. However, regarding the lower limit thereof, the pH is preferably 1 or greater, more preferably 2 or greater, even more preferably 3 or greater, and most preferably 4 or greater. Regarding the upper limit thereof, the pH is preferably 6 or less, more preferably 5 or less, and most preferably 4 or less. By employing such a first washing liquid, particles adsorbing a nucleic acid can be efficiently washed with the first washing liquid even when the nucleic acid or the particles adsorbing the nucleic acid are in contact with an alcohol-containing solution on the upstream side of the first plug, that is, even when the nucleic acid is extracted with an alcohol-containing adsorbent liquid to be described later or even when the particles adsorbing the nucleic acid are washed with an alcohol-containing washing liquid. In addition, it is possible to prevent the alcohol from being brought downstream, which is the so-called carry-over of alcohol.

The volume of the second plug 20 is not particularly limited, and can be appropriately set with an amount of particles adsorbing a nucleic acid as an index. For example, when the volume of the particles is 0.5 µL, it is sufficient that the volume of the second plug 20 is 10 µL or greater, and it is preferably 20 µL to 50 µL, and more preferably 20 µL to 30 µL. When the volume of the second plug 20 is within this range, washing of the particles can be sufficiently performed when the volume of the particles is 0.5 µL. The greater the volume of the second plug 20, the more preferable to wash the particles. However, the volume of the second plug 20 can be appropriately set in consideration of the length and thickness of the tube portion 100, the length of the second plug 20 in the longitudinal direction of the tube portion 100 depending thereon, and the like.

The second plug 20 may be partitioned by oil plugs so as to be formed of a plurality of plugs. When the second plug 20 is formed of a plurality of plugs partitioned by oil plugs, a plurality of first washing liquid plugs is formed. Accordingly, it is preferable that the second plug 20 be partitioned by oil plugs since when a washing target is a water-soluble substance, a concentration of the water-soluble substance reached by a partitioned first washing liquid is lower than a concentration of the water-soluble substance reached by an unpartitioned first washing liquid having the same volume. The number of plugs into which the second plug 20 is to be partitioned is arbitrarily set. When a washing target is a water-soluble substance, and for example, the second plug 20 is partitioned into two plugs having equal volumes, a calculated concentration of the water-soluble substance can be reduced up to 1/4 of a concentration of a case in which the second plug 20 is not partitioned. The number of plugs into which the second plug 20 is to be partitioned can be appropriately set in consideration of, for example, the length of the tube portion 100, the washing target, and the like.

### 1.4. Fourth Plug

The fourth plug 40 is disposed at a position between the third plug 30 and the fifth plug 50 in the tube portion 100. The fourth plug 40 is formed of an eluate for eluting a nucleic acid from fine particles to which the nucleic acid is bonded.

As the eluate, purified water such as sterilized water, distilled water, or ion-exchanged water, or a buffer can be used. When the eluate is water or an aqueous solution, the nucleic acid adsorbed to particles can be eluted by immersing the particles adsorbing the nucleic acid in the eluate. The eluate is a liquid which does not mix with both the oil of the third plug 30 and the oil of the fifth plug 50.

For a reverse transcription reaction, the eluate may contain a reverse transcriptase, dNTP, and a primer for a reverse transcriptase (oligonucleotide), and for a polymerase reaction, the eluate may further contain a DNA polymerase and a primer for a DNA polymerase (oligonucleotide). The eluate may contain a probe for real-time PCR such as a TaqMan probe, a molecular beacon probe, or a cycling probe, or a fluorescent dye for an intercalator such as SYBR Green. Furthermore, the eluate preferably contains bovine serum albumin (BSA) or gelatin as a reaction inhibition preventing agent. Water is preferably used as a solvent, and it is more preferable that an organic solvent such as ethanol and isopropyl alcohol and a chaotropic substance be not substantially contained. In addition, the eluate preferably contains a salt so as to serve as a buffer solution for a reverse transcriptase and/or a buffer solution for a DNA polymerase. The salt for providing the buffer solution is not particularly limited as long as it does not inhibit an enzymatic reaction, but a salt such as Tris, HEPES, PIPES, or a phosphate is preferably used. The reverse transcriptase is not particularly limited, and a reverse transcriptase derived from, for example, avian myeloblast virus, Ras associated virus type 2, mouse molony murine leukemia virus, or human immunodefficiency virus type 1, can be used. However, a heat-resistant enzyme is preferred. The DNA polymerase is also not particularly limited, but is preferably a heat-resistive enzyme or an enzyme for PCR. There are a great number of commercially available products such as Taq polymerase, Tfi polymerase, Tth polymerase, and a modified form thereof, and a DNA polymerase capable of performing hot start is preferred.

The primer for a DNA polymerase can easily determine an appropriate arrangement of DNA to be detected. Usually, in order to amplify one kind of DNA, a primer pair of a 5'-side primer and a 3'-side primer may be included. In order to amplify a plurality of kinds of DNA, a plurality of kinds of primer pairs labeled with different fluorescent dyes may be included to deal with multiplex PCR. In that case, a plurality of TaqMan probes may be appropriately provided.

The concentrations of the dNTP and the salt contained in the reaction liquid may be set to concentrations suitable for the enzyme to be used. The concentration of the dNTP may be set generally to 10 µM to 1,000 µM, and preferably 100 µM to 500 µM, the concentration of Mg²⁺ may be set to 1 mM to 100 mM, and preferably 5 mM to 10 mM, and the concentration of Cl⁻ may be set to 1 mM to 2,000 mM, and preferably 200 mM to 700 mM. The total ion concentration is not particularly limited, but may be higher than 50 mM, preferably higher than 100 mM, more preferably higher than 120 mM, even more preferably higher than 150 mM, and yet more preferably higher than 200 mM. The upper limit thereof is preferably 500 mM or lower, more preferably 300 mM or lower, and even more preferably 200 mM or lower. Each oligonucleotide for the primer is used in an amount of 0.1 µM to 10 µM, and preferably 0.1 µM to 1 µM. When the concentration of the BSA or the gelatin is 1 mg/mL or less, a reaction inhibition prevention effect is reduced, and when the concentration of the BSA or the gelatin is 10 mg/mL or greater, the reverse transcription reaction or the subsequent enzymatic reaction may be inhibited. Accordingly, the concentration of the BSA or the gelatin is preferably 1 mg/mL to 10 mg/mL. In the case of using gelatin, the gelatin may be derived from, for example, cattle skin, pig skin, or cattle bone, but the origin thereof is not particularly limited thereto. If the gelatin dissolves rarely, it may be heated to facilitate dissolution.

The volume of the eluate plug 40 can be appropriately set with an amount of particles adsorbing a nucleic acid, an amount of the reaction liquid used when performing a nucleic acid amplification reaction, or the like as an index. For example, when the volume of the particles is 0.5 µL, it is sufficient that the volume of the eluate plug 40 is 0.5 µL or greater, and for example, when a nucleic acid amplification reaction is performed using a rotation-type PCR apparatus of which the principle is disclosed in JP-A-2012-115208, the smaller the volume of the eluate plug 40 to be introduced to a nucleic acid amplification reaction container, the more preferable, and thus the volume of the eluate plug 40 is preferably 5 µL or less. That is, the volume of the eluate plug 40 is preferably 0.5 µL to 5 µL, and more preferably 1 µL to 3 µL. When the volume of the eluate plug is within these ranges, elution of the nucleic acid from the particles can be sufficiently performed even when the volume of the fine particles is 0.5 µL. Thus, the nucleic acid amplification can be rapidly performed even when the nucleic acid amplification reaction is performed using the rotation-type PCR apparatus.

### 1.5. Configuration of Nucleic Acid Extraction Device, etc.

The nucleic acid extraction device of this embodiment has the tube portion 100, the first plug 10, the second plug 20, the third plug 30, the fourth plug 40, and the fifth plug 50. However, it may also include a configuration with other functions added thereto. The nucleic acid extraction device of this embodiment may include a combination of configurations to be described below, and modifications of the configurations.

### 1.5.1. End Portion of Tube Portion

Fig. 2 is a diagram schematically illustrating a nucleic acid extraction device 1010 which is a modification example of the nucleic acid extraction device. The nucleic acid extraction device of this embodiment may have, for example, an open end on the side of a fifth plug 50 of a tube portion 100. That is, as illustrated in Fig. 2, in the nucleic acid extraction device 1010, the end on the side of the fifth plug 50 of the tube portion 100 is open. According to the nucleic acid extraction device 1010, the fifth plug 50 and a fourth plug 40 can be discharged in order by applying a pressure to the inside of the tube portion 100 from a side of a first plug 10 of the tube portion 100. Accordingly, an eluate (fourth plug 40) containing a target nucleic acid can be easily dispensed to, for example, a reaction container for PCR using the nucleic acid extraction device 1010.

### 1.5.2. Cock

Fig. 3 is a diagram schematically illustrating a nucleic acid extraction device 1020 which is a modification example of the nucleic acid extraction device. The nucleic acid extraction device of this embodiment may further have, for example, a detachable cock 110 to seal an end on the side of a fifth plug 50 of a tube portion 100 as illustrated in Fig. 3. The cock 110 can be made of, for example, a rubber, an elastomer, or a polymer. When the tube portion 100 is sealed by the cock 110, the cock 110 may be in contact with the fifth plug 50, or a gas such as air may be disposed between the fifth plug 50 and the cock 110. Although the cock 110 is detachable, the mechanism thereof is not particularly limited. The example of Fig. 3 illustrates an aspect in which a part of the cock 110 is inserted into the tube portion 100 and fixed, but the cock 110 may have a cap form.

When the cock 110 is removed in the nucleic acid extraction device 1020, the end on the side of the fifth plug 50 of the tube portion 100 is opened, and thus the aspect of the nucleic acid extraction device 1010 of Fig. 2 is provided, and an eluate (fourth plug 40) containing a target nucleic acid can be easily dispensed to, for example, a reaction container for PCR using the nucleic acid extraction device 1020. When the cock 110 seals the end on the side of the fifth plug 50 of the tube portion 100 (Fig. 3), an effect of suppressing the movement of each plug in the tube portion 100 is obtained. Thus, for example, when particles are moved in the tube portion 100, the movement of the plug with the movement of the particles can be suppressed.

### 1.5.3. Container

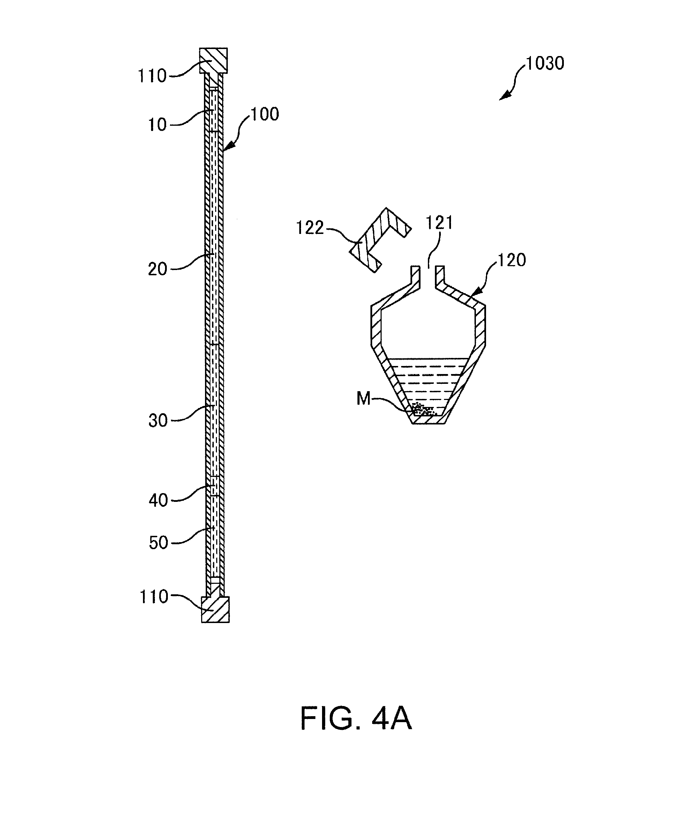

Fig. 4A is a diagram schematically illustrating a nucleic acid extraction device 1030 which is an example of the configuration of the nucleic acid extraction device. As illustrated in Fig. 4A, the nucleic acid extraction device 1030 further has a detachable container 120 which can be connected to an upstream end on the side of a first plug 10 of a tube portion 100 by internal communication.

The container 120 can be formed as a separate member. The container 120 can accommodate a liquid therein. The container 120 has an opening 121 through which a liquid and a solid can be put in and out. The example of Fig. 4A illustrates an aspect in which the opening 121 of the container 120 is connected to the end on the side of the first plug 10 of the tube portion 100 by internal communication. The container 120 may have a plurality of openings 121. In this case, an aspect in which one opening 121 is connected to the end on the side of the first plug 10 of the tube portion 100 by internal communication may be employed.

The internal volume of the container 120 is not particularly limited. However, it can be set to 0.1 mL to 100 mL. If necessary, the opening 121 of the container 120 may have such a structure as to be sealed by a lid 122. The material of the container 120 is not particularly limited, and a polymer, a metal, and the like can be used.

The opening 121 of the container 120 can be connected to the end on the side of the first plug 10 of the tube portion 100. However, the connection between the container 120 and the tube portion 100 is not particularly limited as long as the contents do not leak therefrom. When the container 120 and the tube portion 100 are connected to each other, the inside of the container 120 and the inside of the tube portion 100 can be allowed to communicate with each other. If necessary, the container 120 can be detached from the tube portion 100.

As in the nucleic acid extraction device 1030, by providing the container 120, for example, particles, an adsorbent liquid, and a sample can be accommodated in the container 120 and a nucleic acid can be adsorbed to the particles. Thereafter, when the container 120 is connected to the end on the side of the first plug 10 of the tube portion 100, the particles can be easily introduced from the side of the first plug 10 of the tube portion 100 into the tube portion 100.

The adsorbent liquid is a liquid which allows a nucleic acid to be adsorbed to fine particles (for example, magnetic particles M) serving as a nucleic acid binding solid phase carrier. The adsorbent liquid is not particularly limited as long as it contains a chaotropic substance, but a surfactant may be contained therein for the purpose of disrupting cell membranes or denaturing proteins contained in cells. This surfactant is not particularly limited as long as it is generally used for extracting a nucleic acid from cells or the like. Specific examples thereof include nonionic surfactants such as Triton surfactants, e.g., Triton-X and Tween surfactants, e. g. , Tween 20, and anionic surfactants such as sodium N-lauroylsarcosine (SDS). However, it is particularly preferable to use a nonionic surfactant in an amount of 0.1% to 2% by volume. Furthermore, the adsorbent liquid preferably contains a reducing agent such as 2-mercaptoethanol or dithiothreitol. The adsorbent liquid may be a buffer solution, and is preferably has a neutral pH ranging from 6 to 8. In consideration of this, specifically, the adsorbent liquid preferably contains 3 M to 7 M of a guanidine salt, 0% to 5% by volume of a nonionic surfactant, 0 mM to 0.2 mM of EDTA, 0 M to 0.2 M of a reducing agent, and the like.

The chaotropic substance is not particularly limited as long as it generates chaotropic ions (monovalent anions having a large ionic radius) in an aqueous solution, acts to increase the water solubility of hydrophobic molecules, and contributes to the adsorption of a nucleic acid to fine particles. Specific examples thereof include guanidine thiocyanate, guanidine hydrochloride, sodium iodide, potassium iodide, and sodium perchlorate. Among these, guanidine thiocyanate or guanidine hydrochloride which strongly acts on protein denaturation is preferred. The concentrations of such chaotropic substances used vary among the respective substances, and for example, when guanidine thiocyanate is used, the concentration used thereof is preferably in a range of 3 M to 5.5 M, and when guanidine hydrochloride is used, the concentration used thereof is preferably 5 M or greater.

This adsorbent liquid preferably contains an alcohol or acetonitrile. In this case, the concentration of the alcohol or the like is not particularly limited. However, regarding the lower limit thereof, the concentration may be 10% by volume or higher, 20% by volume or higher, or 30% by volume or higher, and is most preferably 40% by volume or higher. Regarding the upper limit thereof, the concentration may be 80% by volume or lower, 70% by volume or lower, or 60% by volume or lower, and is most preferably 50% by volume or lower. The kind of the alcohol is not particularly limited, and methanol, ethanol, propanol, and the like can be exemplified. The effect of adsorbing the nucleic acid to the particles is increased by adding the alcohol or the like to the adsorbent liquid, and thus considering only the nucleic acid extraction device, it is possible to increase nucleic acid extraction efficiency.

The container 120 can be shaken in a state of being disconnected from the tube portion 100 to sufficiently stir the liquid in the container 120. Accordingly, the nucleic acid can be rapidly adsorbed to the particles. The container 120 may have the lid 122 to seal the opening 121. Furthermore, by appropriately changing the amount of the sample to be introduced to the container 120 and the volume of the liquid (particularly, fourth plug 40) in the tube portion 100, the nucleic acid in the sample can be quantitatively concentrated in the eluate of the fourth plug 40.

When a flexible material such as a rubber, an elastomer, or a polymer is selected as the material of the container 120, the inside of the tube portion 100 can be pressurized by deforming the container 120 in a state in which the container 120 is connected to the tube portion 100. Thus, when the eluate of the fourth plug 40 is discharged from an end on the side of a fifth plug 50 of the tube portion 100, the pressure is easily applied from the side of the first plug 10 of the tube portion 100. Accordingly, the eluate can be dispensed to, for example, a reaction container for PCR.

### 1.5.4. Liquid Reservoir

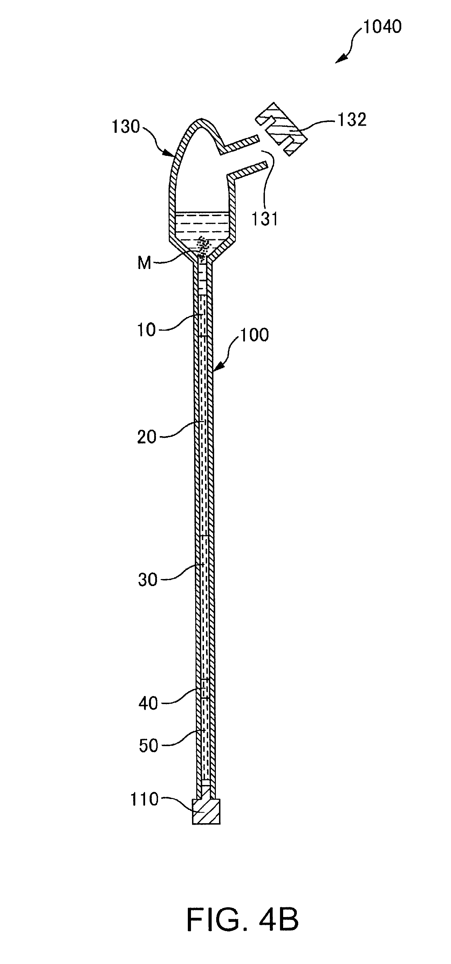

Fig. 4B is a diagram schematically illustrating a nucleic acid extraction device 1040 which is an example of the configuration of the nucleic acid extraction device. As illustrated in Fig. 4B, the nucleic acid extraction device 1040 has a liquid reservoir 130 which is formed at an end on the side of a first plug 10 of a tube portion 100 to communicate with the tube portion 100. The inside of the liquid reservoir 130 and the inside of the tube portion 100 communicate with each other.

The liquid reservoir 130 can accommodate a liquid therein. The liquid reservoir 130 has an opening 131 through which a substance can be introduced from the outside to the inside of the liquid reservoir 130. The position at which the opening 131 is formed in the liquid reservoir 130 is not particularly limited. The liquid reservoir 130 may have a plurality of openings 131. The internal volume of the liquid reservoir 130 is not particularly limited. However, it can be set to 0. 1 mL to 100 mL. The material of the liquid reservoir 130 is not particularly limited, and a polymer, a metal, or the like can be used. The material of the liquid reservoir 130 may be the same as that of the tube portion 100.

As in the nucleic acid extraction device 1040, by providing the liquid reservoir 130, for example, particles, an adsorbent liquid, and a sample can be accommodated in the liquid reservoir 130 and a nucleic acid can be adsorbed to the particles. The particles can be easily introduced into the tube portion 100 from the side of the first plug 10 of the tube portion 100.

In addition, the liquid reservoir 130 can be shaken together with the tube portion 100 to sufficiently stir the liquid in the liquid reservoir 130. Accordingly, the nucleic acid can be rapidly adsorbed to the particles. Furthermore, by appropriately changing the amount of the sample to be introduced to the liquid reservoir 130 and the volume of the liquid in the tube portion 100, the nucleic acid in the sample can be quantitatively concentrated in an eluate.

When the liquid reservoir 130 is provided as in the nucleic acid extraction device 1040, a detachable lid 132 may be further provided to seal the opening 131 of the liquid reservoir 130. When a flexible material such as a rubber, an elastomer, or a polymer is selected as the material of the liquid reservoir 130, the inside of the tube portion 100 can be pressurized by deforming the liquid reservoir 130 in a state in which the lid 132 is mounted on the liquid reservoir 130.

Thus, when the eluate of a fourth plug 40 in which the nucleic acid is eluted is discharged from an end on the side of a fifth plug 50 of the tube portion 100, the pressure can be easily applied from the side of the first plug 10 of the tube portion 100. Accordingly, it is possible to perform the processing ranging from a process of introducing a sample to the container 120 to a process of easily dispensing an eluate to, for example, a reaction container for PCR. In addition, when mounting the lid 132, it is possible to suppress liquid leakage when the liquid reservoir 130 is shaken together with the tube portion 100. Thus, the efficiency of adsorbing the nucleic acid to particles can be improved.

### 1.5.5. Sixth Plug and Seventh Plug

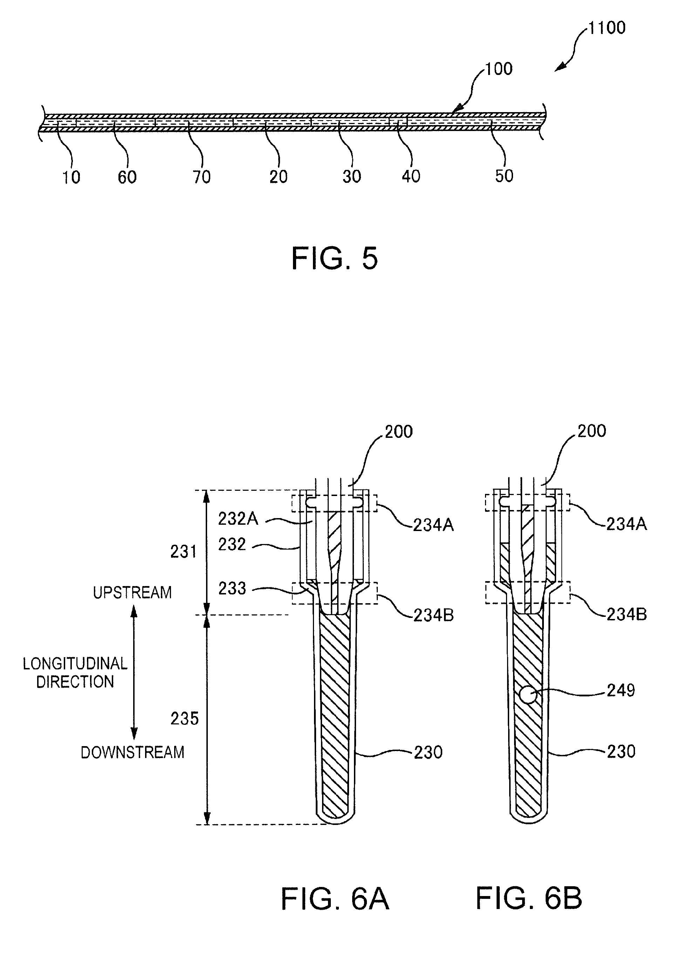

The nucleic acid extraction device of this embodiment may have a sixth plug and a seventh plug in the tube portion. Fig. 5 is a diagram schematically illustrating a nucleic acid extraction device 1100 having a sixth plug 60 and a seventh plug 70 in a tube portion 100.

The nucleic acid extraction device 1100 has a configuration in which between a first plug 10 and a second plug 20 in the tube portion 100 of the above-described nucleic acid extraction device, the sixth plug 60 formed of a second washing liquid which is phase-separated from an oil when mixing therewith and is provided to wash fine particles to which a nucleic acid is bonded, and the seventh plug 70 formed of a fourth oil are added in order from the side of the first plug 10.

The sixth plug 60 is formed of a second washing liquid. The second washing liquid may be a liquid which is phase-separated from any of the oil of the first plug 10 and the oil of the seventh plug 70 when mixing therewith. The second liquid is preferably water or an aqueous solution with a low salt concentration. In the case of the aqueous solution with a low salt concentration, the second liquid is preferably a buffer solution. The salt concentration in the aqueous solution with a low salt concentration is preferably 100 mM or lower, more preferably 50 mM or lower, and most preferably 10 mM or lower. The lower limit of the salt concentration in the aqueous solution with a low salt concentration is not particularly limited, but is preferably 0.1 mM or higher, more preferably 0.5 mM or higher, and most preferably 1 mM or higher. In addition, this solution may contain a surfactant such as Triton, Tween, or SDS, and the pH thereof is not particularly limited. The salt for forming the buffer solution is not particularly limited, but a salt such as Tris, HEPES, PIPES, or a phosphate is preferably used. A chaotropic substance may be contained.

Furthermore, this washing liquid preferably contains an alcohol to increase the washing effect. Particularly, in order to increase the effect provided by using an acidic solution as the first washing liquid, at least any of the eluate and the second washing liquid preferably contains an alcohol. In this case, the concentration of the alcohol is not particularly limited. However, regarding the lower limit thereof, the concentration may be 50% by volume or higher or 60% by volume or higher, and is most preferably 70% by volume or higher. Regarding the upper limit of the concentration of the alcohol, the concentration may be 90% by volume or lower or 80% by volume or lower, and is most preferably 70% by volume or lower. The kind of the alcohol is not particularly limited, and methanol, ethanol, propanol, acetonitrile, and the like can be exemplified. From the viewpoint of washing the nucleic acid and the particles adsorbing the nucleic acid, the washing effect can be increased when at least one of the above-described adsorbent liquid and the second washing liquid contains an alcohol. Both of the adsorbent liquid and the second washing liquid preferably contain an alcohol since the washing effect can be further increased.

The volume of the sixth plug 60 is not particularly limited, and can be appropriately set with an amount of particles adsorbing a nucleic acid as an index. For example, when the volume of the particles is 0.5 µL, it is sufficient that the volume of the sixth plug 60 is 10 µL or greater, and it is preferably 20 µL to 50 µL, and more preferably 20 µL to 30 µL. When the volume of the sixth plug 60 is within this range, washing of the particles can be sufficiently performed when the volume of the particles is 0.5 µL. The greater the volume of the sixth plug 60, the more preferable to wash the particles. However, the volume of the sixth plug 60 can be appropriately set in consideration of the length and thickness of the tube portion 100, the length of the sixth plug 60 in the longitudinal direction of the tube portion 100 depending thereon, and the like.

The sixth plug 60 may be partitioned by oil plugs so as to be formed of a plurality of plugs. When the sixth plug 60 is formed of a plurality of plugs partitioned by oil plugs, the configurations of the washing liquids of the plugs may be the same as or different from each other, and are not particularly limited. However, as described above, these preferably contain an alcohol.

The seventh plug 70 is formed of an oil which does not mix with the liquids of the sixth plug 60 and the second plug 20 next thereto. The oil of the seventh plug 70 may be the same kind of oil as or a different kind of oil from the oils of the first plug 10, the third plug 30, and the fifth plug 50. Examples of the oil include those exemplified in the case of the first plug 10 and the like.

It is preferable that bubbles and other liquids do not exist in the seventh plug 70. However, as long as particles adsorbing a nucleic acid can pass through the seventh plug 70, bubbles and other liquids may exist. In addition, it is preferable that bubbles and other liquids do not exist between the second plug 20 and the sixth plug 60 next to the seventh plug 70. However, as long as particles adsorbing a nucleic acid can be moved in the tube portion 100, bubbles and other liquids may exist.

The length of the seventh plug 70 in the longitudinal direction of the tube portion 100 is not particularly limited within a range in which the plug can be formed. The length of the seventh plug 70 in the longitudinal direction of the tube portion 100 is specifically 1 mm to 50 mm. In order to keep a moving distance of particles from increasing excessively, the length is preferably 1 mm to 30 mm, and more preferably 5 mm to 20 mm.

In addition, the seventh plug 70 functions to suppress the mixing of the second washing liquid (sixth plug 60) and the first washing liquid (second plug 20). In addition, when the seventh plug 70 is formed of an oil having a higher viscosity, a "wiping effect" of the oil can be increased when particles are moved in an interface with the second washing liquid (sixth plug 60). Accordingly, when particles are moved from the second washing liquid plug which is the sixth plug 60 to the seventh oil plug 70, it is possible to make it harder for a water-soluble component adhering to the particles to be brought into the seventh plug 70.

According to the nucleic acid extraction device 1100, particles adsorbing a nucleic acid can be washed in the second plug 20 and the sixth plug 60. Therefore, efficiency of washing the particles can be further increased.

In the nucleic acid extraction device 1100, the second washing liquid of the sixth plug 60 may contain a chaotropic agent. For example, when the second washing liquid contains guanidinium hydrochloride, it is possible to wash the particles while maintaining or strengthening the adsorption of the nucleic acid adsorbed to the particles in the sixth plug 60. The concentration when the sixth plug 60 contains guanidinium hydrochloride can be set to, for example, 3 mol/L to 10 mol/L, and preferably 5 mol/L to 8 mol/L. When the concentration of the guanidinium hydrochloride is within this range, it is possible to wash other foreign substances while more stably adsorbing the nucleic acid adsorbed to the particles.

When an acidic solution is used as the first washing liquid of the second plug 20 as described above, the alcohol adsorbed to the particles in the sixth plug 60 can be efficiently subjected to washing in the second plug 20, and thus it is possible to reduce the bringing of the alcohol into the eluate or into a reverse transcription reaction liquid or a nucleic acid amplification reaction liquid to be used later.

It should be easily understood that the nucleic acid extraction device 1100 having the sixth plug 60 and the seventh plug 70 in the tube portion 100 may also have a configuration with the above-described cock, container, liquid reservoir, and the like added thereto, and thus similar effects to those described above are obtained.

### 1.5.6. Nucleic Acid Amplification Reaction Container

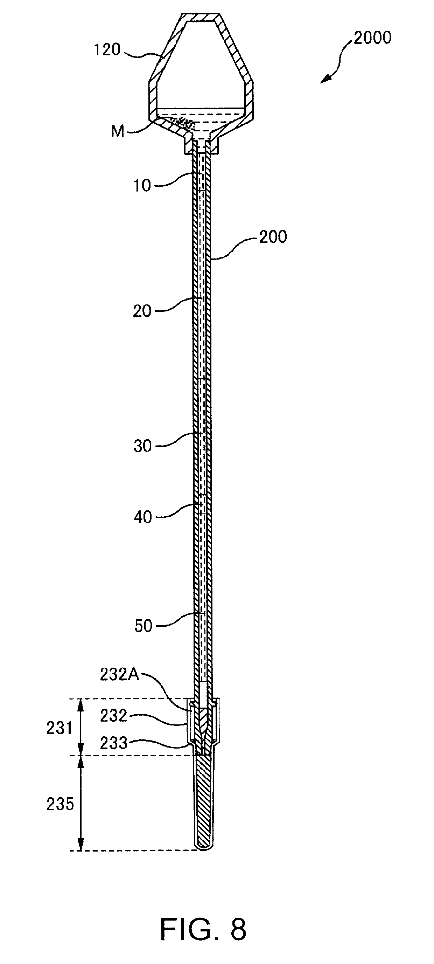

The nucleic acid extraction device according to the invention may be configured as a nucleic acid amplification reaction cartridge provided with a nucleic acid amplification reaction container which communicates with a downstream end of a tube and contains an oil. Figs. 6A and 6B illustrate examples of a configuration of the nucleic acid amplification reaction container, and Fig. 8 illustrates an example of an overall configuration of the nucleic acid amplification reaction cartridge.

Fig. 6A is a diagram illustrating an initial state. Fig. 6B is a diagram illustrating a state after an eluate is pushed out of a tube 200. A nucleic acid amplification reaction container 230 is a container which receives the liquid pushed out of the tube 200, and is a container which accommodates an eluate 249 during a thermal cycle treatment.

The nucleic acid amplification reaction container 230 has a seal forming portion 231 and a flow channel forming portion 235. The seal forming portion 231 is a part into which the tube 200 is inserted, and is a portion which suppresses the leakage of the oil overflowing from the flow channel forming portion 235 to the outside. The flow channel forming portion 235 is a part on the downstream side of the seal forming portion 231, and is a portion which forms a flow channel in which the droplet-like eluate 249 moves. The nucleic acid amplification reaction container 230 is fixed to the tube 200 in two places, i.e., an upper sealing portion 234A and a lower sealing portion 234B of the seal forming portion 231.

The seal forming portion 231 has an oil receiving portion 232 and a stage portion 233.

The oil receiving portion 232 is a tubular portion and functions as a reservoir which receives the oil overflowing from the flow channel forming portion 235. There is a gap between an internal wall of the oil receiving portion 232 and an external wall of the tube 200, and this gap serves as an oil receiving space 232A which receives the oil overflowing from the flow channel forming portion 235.

The upper sealing portion 234A is formed through the contact of the internal wall on the upstream side of the oil receiving portion 232 with an annular convex portion of the tube 200. The upper sealing portion 234A is a seal which suppresses the leakage of the oil in the oil receiving space 232A to the outside while permitting the passage of air. The upper sealing portion 234A has a vent hole formed to function to the extent that the oil does not leak due to the surface tension of the oil. The vent hole of the upper sealing portion 234A may be a gap between the convex portion of the tube 200 and the internal wall of the oil receiving portion 232, or a hole, a groove, or a notch formed in the convex portion of the tube 200. The upper sealing portion 234A may be made of an oil absorbing material which absorbs an oil.

The stage portion 233 is a portion with a stage provided on the downstream side of the oil receiving portion 232. The downstream portion of the stage portion 233 has an internal diameter smaller than an internal diameter of the oil receiving portion 232. An internal wall of the stage portion 233 is brought into contact with an external wall on the downstream side of the tube 200. The lower sealing portion 234B is formed through the contact of the internal wall of the stage portion 233 with the external wall of the tube 200. The lower sealing portion 234B is a seal which resists the flowing of the oil in the flow channel forming portion 235 to the oil receiving space 232A while permitting the flowing. Since the pressure in the flow channel forming portion 235 is increased compared to the outside pressure due to a pressure loss in the lower sealing portion 234B, bubbles are rarely generated in the liquid in the flow channel forming portion 235 even when the liquid in the flow channel forming portion 235 is heated during a thermal cycle treatment.

The flow channel forming portion 235 is a tubular portion, and is a container which forms a flow channel in which the droplet-like eluate 249 moves. The flow channel forming portion 235 is filled with an oil. The upstream side of the flow channel forming portion 235 is closed with an end of the tube 200, and the end of the tube 200 is open toward the flow channel forming portion 235. The flow channel forming portion 235 has an internal diameter which is larger than an internal diameter of the tube 200 and is also larger than an external diameter of the eluate 249 when the eluate 249 has a spherical shape. An internal wall of the flow channel forming portion 235 preferably has water repellency to the extent that the water-soluble eluate 249 does not adhere thereto.

As illustrated in Fig. 6A, the flow channel forming portion 235 of the nucleic acid amplification reaction container 230 is filled with an oil in an initial state. The interface of the oil is positioned on the relatively downstream side of the oil receiving space 232A.

As illustrated in Fig. 6B, even when the eluate 249 is pushed out of the tube 200, no gas flows into the flow channel forming portion 235 since the flow channel forming portion 235 is filled with the oil in advance.

Before the eluate 249 is pushed out of the tube 200, first, the oil in an oil plug disposed at the farthest downstream position in the tube 200 flows into the flow channel forming portion 235, and the flowing oil flows from the flow channel forming portion 235 to the oil receiving space 232A, whereby the interface of the oil in the oil receiving space 232A is increased. At this time, the pressure of the liquid in the flow channel forming portion 235 is increased due to a pressure loss in the lower sealing portion 234B. After the oil plug disposed at the farthest downstream position is pushed out of the tube 200, the eluate 249 flows into the flow channel forming portion 235 from the tube 200. When the eluate plug 249 contains a nucleic acid to be amplified and a reagent for performing a nucleic acid amplification reaction, the inflowing eluate plug 249 becomes like droplets and is used in PCR as is.

Such an integral-type nucleic acid amplification reaction cartridge can be appropriately used for the rotation-type PCR apparatus of which the principle is disclosed in JP-A-2012-115208.

### 2. Nucleic Acid Extraction Kit

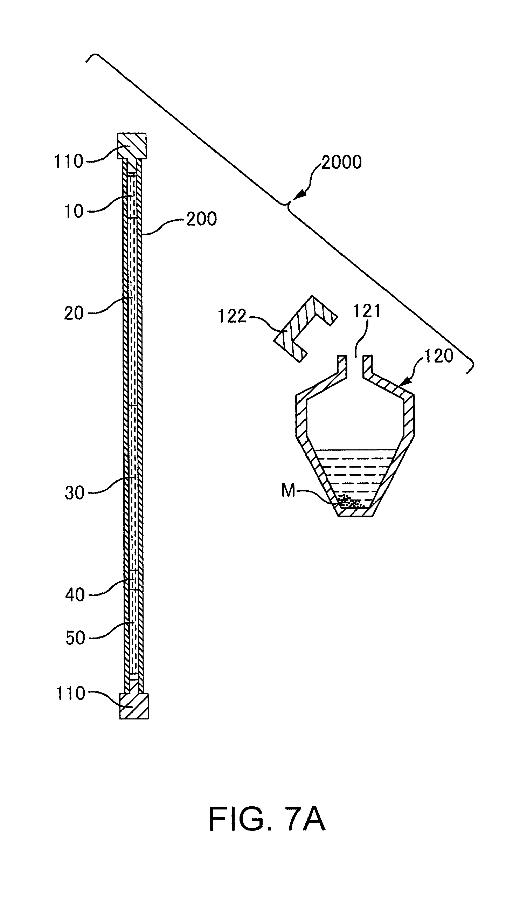

Fig. 7A is a schematic diagram illustrating an example of a nucleic acid extraction kit of this embodiment. A nucleic acid extraction kit 2000 illustrated in Fig. 7A includes components constituting the main part of the above-described nucleic acid extraction device. Similar configurations to those described in the clause "1. Nucleic Acid Extraction Device" will be denoted by the same reference numerals, and detailed description thereof will be omitted.

The nucleic acid extraction kit 2000 of this embodiment includes a tube 200 in which a first plug 10 formed of an oil, a second plug 20 formed of a first washing liquid which does not mix with an oil, a third plug 30 formed of an oil, a fourth plug 40 formed of an eluate which does not mix with an oil, and a fifth plug 50 formed of an oil are arranged in this order, and a container 120 which can be connected to an end on the side of the first plug 10 of the tube 200 by internal communication.

The tube 200 has an aspect in which both ends of the tube portion 100 of the nucleic acid extraction device 1000 are open. The tube 200 has a cavity therein and has a tubular shape in which a liquid can be allowed to flow in a longitudinal direction in the cavity. The internal shape, external shape, size, properties, material, and the like of the tube 200 are similar to those of the tube portion 100 of the nucleic acid extraction device 1000. The plugs arranged in the tube 200 are similar to the plugs arranged in the tube portion 100 of the nucleic acid extraction device 1000. Both ends of the tube 200 may be sealed by detachable cocks 110. When both the ends of the tube 200 are sealed by the cocks 110, for example, storage and transfer of the nucleic acid extraction kit 2000 are facilitated. Furthermore, when the cock 110 seals an end on the side of the fifth plug 50 of the tube 200 during the use of the tube 200, the movement of each plug in the tube 200 can be suppressed when particles are moved in the tube 200, and thus washing and extraction can be further facilitated. Since the cock 110 is detachable, the end on the side of the fifth plug 50 of the tube 200 can be opened, and thus the eluate of the fourth plug 40 in which a nucleic acid is eluted is easily discharged from the end on the side of the fifth plug 50 of the tube 200.

The container 120 is similar to the container 120 described in the clause of the nucleic acid extraction device 1000.

In the example of Fig. 7A, both the ends of the tube 200 are sealed by the detachable cocks 110. The nucleic acid extraction kit 2000 may include a lid 122 which detachably seals an opening 121 of the container 120, or the opening 121 of the container 120 may be sealed by the detachable lid 122. The nucleic acid extraction kit 2000 may accommodate some or all of components of an adsorbent liquid in the container 120.

In addition, in the nucleic acid extraction kit 2000, the container 120 may accommodate an adsorbent liquid and magnetic particles. Thus, a process of adsorbing, when a sample is introduced into the container 120, a nucleic acid contained in the sample to the magnetic particles can be performed in the container 120. Accordingly, it is possible to more rapidly perform preprocessing of PCR without the need to provide another container. In addition, in this case, the opening 121 of the container 120 may be sealed by the detachable lid 122 if necessary. The magnetic particles will be described later in detail.

When the container 120 is made of a flexible material as described above, the inside of the tube 200 can be pressurized by deforming the container 120 in a state in which the container 120 is connected to the tube 200. Thus, when the eluate of the fourth plug 40 in which the nucleic acid is eluted is discharged from the end on the side of the fifth plug 50 of the tube 200, the pressure can be easily applied from the side of the first plug 10 of the tube 200. Accordingly, it is possible to easily dispense the eluate to, for example, a reaction container for PCR.

The nucleic acid extraction kit 2000 may include other configurations such as a cock, a lid, an instruction manual, a reagent, and a case, other than the tube 200 and the container 120. Here, an example has been shown in which five plugs are arranged in the tube 200. However, as described in the clause "1.6. Nucleic Acid Extraction Device", it should be easily understood that if necessary, other plugs such as the sixth plug 60 and the seventh plug 70 may be arranged in the tube 200 (tube portion 100).

The nucleic acid extraction kit 2000 of this embodiment has the container 120 which can be connected to the end on the side of the first plug 10 of the tube 200 by internal communication. Accordingly, when particles and a sample are accommodated in the container 120, the nucleic acid can be adsorbed to the particles, and thus the particles can be easily introduced into the tube 200 from the side of the first plug of the tube 200 when the container 120 is connected to the end on the side of the first plug 10 of the tube 200. In addition, since the nucleic acid extraction kit 2000 of this embodiment has the container 120, the container 120 can be shaken, and thus the liquid in the container 120 can be sufficiently stirred. Accordingly, the nucleic acid can be rapidly adsorbed to the particles.

When connecting the container 120 to the tube 200, the particles adsorbing the nucleic acid are easily moved up to the fourth plug 40 by introducing the particles from the end on the side of the first plug 10 of the tube 200. Accordingly, the nucleic acid can be easily extracted in a very short time. The nucleic acid extraction kit 2000 can obtain an eluate containing the nucleic acid of high purity by moving the particles adsorbing the nucleic acid in the tube 200. Therefore, according to the nucleic acid extraction kit 2000, the time and effort required for preprocessing for PCR can be significantly reduced.

### 3. Nucleic Acid Extraction Method

All of the nucleic acid extraction device, the nucleic acid extraction kit, and the modifications thereof, which have been described above, and a nucleic acid extraction apparatus to be described later can be appropriately used in a nucleic acid extraction method of this embodiment. Hereinafter, a method using the above-described nucleic acid extraction kit 2000 will be described as an example of the nucleic acid extraction method of this embodiment.

The nucleic acid extraction method of this embodiment includes: a process of introducing a sample containing a nucleic acid to the flexible container 120 accommodating fine particles such as magnetic particles M and an adsorbent liquid; a process of adsorbing the nucleic acid to the magnetic particles M by vibrating the container 120; a process of connecting the container 120 to the end on the side of the first plug 10 of the tube 200, in which the first plug 10 formed of an oil, the second plug 20 formed of a first washing liquid which does not mix with an oil, the third plug 30 formed of an oil, the fourth plug 40 formed of an eluate which does not mix with an oil, and the fifth plug 50 formed of an oil are arranged in this order, by allowing the inside of the container 120 and the inside of the tube 200 to communicate with each other; and a process of passing the magnetic particles M through the tube 200 from the container 120 by applying a magnetic force to move the magnetic particles M up to the position of the fourth plug 40, thereby washing the fine particles adsorbing the nucleic acid with the first washing liquid and eluting the nucleic acid from the fine particles in the eluate of the fourth plug 40.

In the nucleic acid extraction method of this embodiment, various particles (for example, silica particles, polymer particles, magnetic particles, or the like) can be used as long as the particles can adsorb the nucleic acid using an adsorbent liquid and can be moved in the tube 200. However, in an embodiment of the nucleic acid extraction method to be described below, the magnetic particles M which are particles containing a magnetic body and can adsorb the nucleic acid to surfaces of the particles are used. When particles other than the magnetic particles M are moved in the tube, this movement can be performed using, for example, gravity or a potential difference.

In the nucleic acid extraction method of this embodiment, a material transmitting a magnetic force is selected for the container 120 and the tube 200, and a magnetic force is applied from the outside of the container 120 and the tube 200 to move the magnetic particles M in the container 120 and the tube 200.

The sample contains a nucleic acid which becomes a target. Hereinafter, it may be simply referred to as a target nucleic acid. The target nucleic acid is extracted from the sample and eluted to the eluate through the nucleic acid extraction method of this embodiment, and then for example, when the nucleic acid is mRNA, it is reverse-transcribed into cDNA and used as a template of PCR, and when the nucleic acid is cDNA or genomic DNA, it is used as a template of PCR as is. Blood, nasal cavity mucous, oral mucous membrane, various biological samples, a partially purified nucleic acid solution, and the like may be used as the sample.

### 3.1. Process of Introducing Sample to Container

The process of introducing the sample to the container 120 can be performed in such a manner that for example, the sample is attached to a swab and the swab is put from the opening 121 of the container 120 to immerse the swab in the adsorbent liquid. The sample may be introduced from the opening 121 of the container 120 using a pipette or the like. When the sample has a paste form or a solid form, the sample may be attached or fed to an internal wall of the container 120 using a spoon, tweezers, or the like from the opening 121 of the container 120.

### 3.2. Process of Adsorbing Nucleic Acid to Magnetic Particles

The process of adsorbing the nucleic acid is performed by vibrating the container 120. When there is the lid 122 which seals the opening 121 of the container 120, this process can be more efficiently performed by sealing the container 120 using the lid 122. Through this process, the target nucleic acid is adsorbed to surfaces of the magnetic particles M by an action of a chaotropic agent. In this process, a nucleic acid other than the target nucleic acid or protein may be adsorbed to the surfaces of the magnetic particles M.

As the method of vibrating the container 120, an apparatus such as a vortex shaker may be used, or the container 120 may be shaken by operator's hand. A magnetic property of the magnetic particles M may be used to vibrate the container 120 while giving a magnetic field from the outside. The time for vibration of the container 120 can be appropriately set. For example, when the shape of the container 120 is roughly a cylindrical shape having a diameter of approximately 20 mm and a height of approximately 30 mm, stirring is sufficiently performed by shaking and vibrating the container 120 for 10 seconds by hand to adsorb the nucleic acid to the surfaces of the magnetic particles M.

### 3.3. Process of Connecting Container to Tube

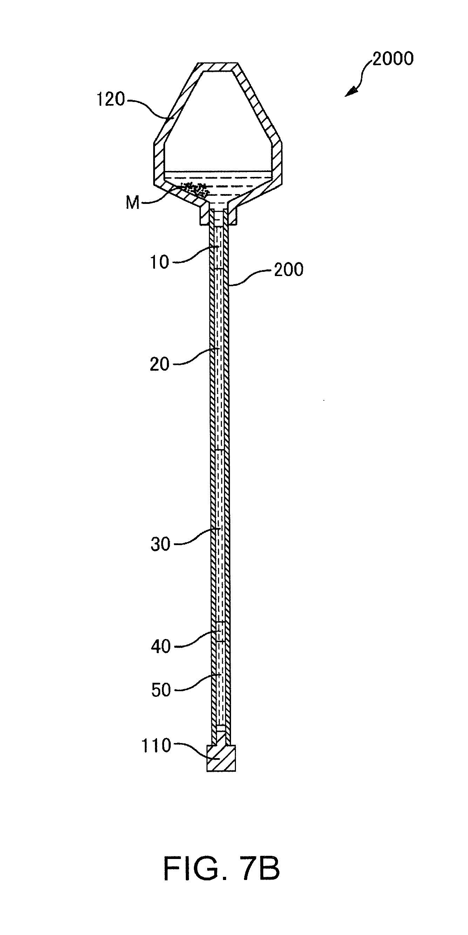

Next, the container 120 is connected to the end on the side of the first plug 10 of the tube 200 as illustrated in Fig. 7B. Each plug in the tube 200 is difficult to move in the tube 200 since the cock 110 on the side of the seventh plug 70 is not removed even when the cock 110 on the side of the first plug 10 is removed. When the cock 110 is attached to the end on the side of the first plug 10 of the tube 200, this process is performed after removing the cock 110. The container 120 and the tube 200 are connected to each other so that the contents do not leak therefrom, and are thus allowed to communicate with each other so that the contents can be allowed to flow between the inside of the container 120 and the inside of the tube 200.

### 3.4. Process of Moving Magnetic Particles

Through the above-described processes, the magnetic particles M adsorbing the nucleic acid in the container 120 enter into a state in which these can be allowed to flow in the tube 200. As the method of introducing the magnetic particles M adsorbing the nucleic acid to the tube 200, a method using gravity or a centrifugal force may be used. The introduction method is not particularly limited, but in this embodiment, the introduction is performed by applying a magnetic force from the outside of the container 120 and the tube 200. The magnetic force can be applied using, for example, a permanent magnet, an electromagnet, or the like. However, it is preferable that the magnetic force be applied using a permanent magnet in view of no generation of heat and the like. When a permanent magnet is used, the introduction may be performed by moving the magnet by operator's hand, or a mechanical device or the like may be used. The magnetic particles M have such a property as to be pulled by a magnetic force. Accordingly, using this property, the relative arrangement between: the container 120 and the tube 200; and the permanent magnet is changed to move the magnetic particles M from the inside of the container 120 to the tube 200. Thus, the magnetic particles M are moved from the first plug 10 to the fourth plug 40 through the plugs in order. A staying time in each plug when the magnetic particles M pass through each plug is not particularly limited, and the magnetic particles M may be moved to reciprocate in the longitudinal direction of the tube 200 in the same plug.

### 3.5. Process of Eluting Nucleic Acid

When the magnetic particles M reach the fourth plug 40, the nucleic acid adsorbed to the magnetic particles M is eluted to the eluate of the fourth plug 40 by the action of the eluate. Through this process, the nucleic acid is eluted from the sample to the eluate, and a state in which the nucleic acid is eluted from the sample is made.

### 3.6. Advantages

According to the nucleic acid extraction method of this embodiment, it is possible to easily extract a nucleic acid in a very short time. In the nucleic acid extraction method of this embodiment, magnetic particles M adsorbing a nucleic acid are moved in the tube 200, and thus it is possible to obtain an eluate containing the nucleic acid of high purity. According to the nucleic acid extraction method of this embodiment, the time and effort required for preprocessing for PCR can be significantly reduced.

### 3.7. Process of Discharging Fourth Plug from Tube

The nucleic acid extraction method of this embodiment may include a process of discharging the fifth plug 50 and the fourth plug 40 from an end of the tube 200 on the side opposite to the end connected to the container 120 by deforming the container 120.

This process can be performed by deforming the container 120 after the process described in the clause "3.5. Process of Eluting Nucleic Acid" . When the fourth plug 40 is discharged, the fifth plug 50 is discharged first. The cock 110 sealing the side of the fifth plug 50 of the tube 200 is removed prior to this process to open the end on the side of the fifth plug 50 of the tube 200.

When an external force is applied to the container 120 to increase the internal pressure and the container 120 is thus deformed, each plug is moved from the side of the first plug 10 to the side of the fifth plug 50 of the tube 200 due to the pressure. Accordingly, the fifth plug 50 and the fourth plug 40 are discharged in order from the end on the side of the fifth plug 50 of the tube 200. The third plug 30 may be discharged. However, the second plug 20 is not permitted to be discharged. In this case, for example, when the volume of the third plug 30 is set to be larger than those of other plugs and the length of the third plug 30 in the longitudinal direction of the tube 200 is increased, the second plug 20 is easily prevented from being discharged.

The fourth plug 40 and the fifth plug 50 are discharged to, for example, a reaction container for PCR. Therefore, the eluate and the oil are dispensed to the reaction container for PCR. Usually, the oil has no influence on PCR, and thus for example, an oil of the same kind as the oil of the fifth plug 50 may be accommodated in advance in the reaction container of PCR. In that case, when this process is performed in a state in which the tip end of the tube 200 is in the oil, the eluate containing the target nucleic acid can be introduced to the reaction container of PCR without the contact of the eluate with the outside air. When the nucleic acid extraction method of this embodiment includes this process, the eluate containing the target nucleic acid can be easily dispensed to, for example, a reaction container for PCR.

In a case in which cDNA amplification is performed using this eluate, when a reaction liquid of a reverse transcription and/or a nucleic acid amplification process contains a large amount of the eluate containing the eluted nucleic acid, the nucleic acid contained in a small amount is easily amplified. Accordingly, the reaction liquids preferably contain 50% by volume or more of the eluate, more preferably 70% by volume or more of the eluate, even more preferably 90% by volume or more of the eluate, and most preferably 100% by volume or more of the eluate, that is, a reagent for a reaction is most preferably added in advance to the eluate for eluting the nucleic acid.

Usually, as the ratio of the eluate in the reaction liquid is increased, the bringing of the ethanol contained in the solution which is used immediately before the washing process, e.g. , the adsorbent liquid and/or the second washing liquid inhibits the subsequent reverse transcription reaction or nucleic acid amplification reaction, and a final cDNA yield is reduced. However, in the nucleic acid amplification method according to the invention, the yield can be prevented from being reduced by using an acidic solution as the first washing liquid.

### 3.8. Modification Examples

### 3.8.1. Modification of Process of Moving Magnetic Particles

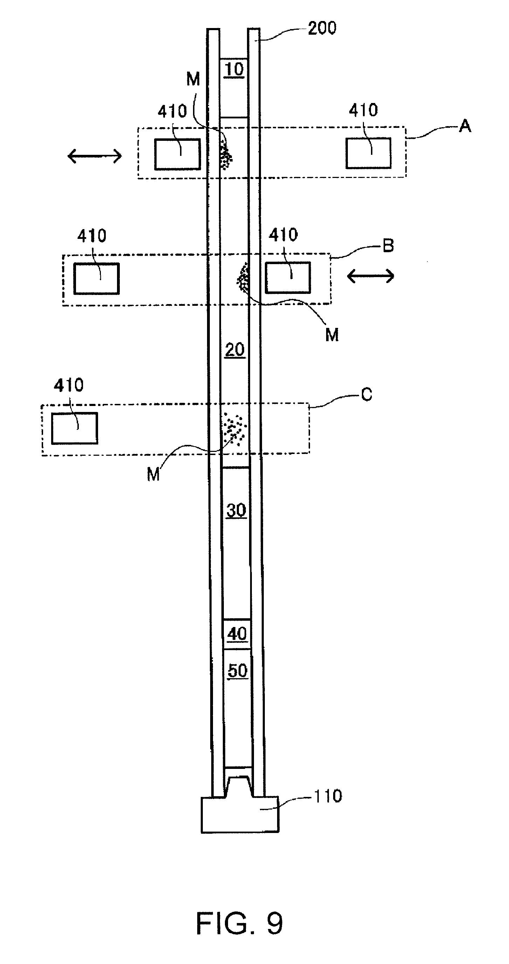

Fig. 9 is a schematic diagram for illustrating a modification of the nucleic acid extraction method of this embodiment.

In the above-described clause "3.4. Process of Moving Magnetic Particles", the magnetic particles M pass through the plugs from the first plug 10 and are moved up to the fourth plug 40 by applying a magnetic force to the magnetic particles M from the outside. However, when the magnetic particles M are moved to the second plug 20, the magnetic particles M may be vibrated in the second plug 20, or may be repeatedly diffused and aggregated by changing the magnetic force applied from the outside. Thus, the effect of washing the magnetic particles M with the first washing liquid of the second plug 20 can be increased.

Specifically, as shown in the boxes A and B of Fig. 9, in a case in which a pair of permanent magnets 410 is used as a unit which applies a magnetic force, when the magnetic particles M are moved from the container 120, pass through the first plug 10, and reach the second plug 20 using the permanent magnets 410, the magnetic particles M can be vibrated in a direction crossing the longitudinal direction of the tube 200 in the second plug 20 when one permanent magnet 410 is moved away from the tube 200 and the other permanent magnet 410 is moved closer from the side opposed to the tube 200 (repetition of the aspects denoted by A and B of Fig. 9). Thus, the effect of washing the magnetic particles M with the first washing liquid of the second plug 20 can be increased. When the second plug 20 is partitioned or the sixth plug 60 is disposed in the tube 200, such washing of the magnetic particles M may also be applied in a plurality of second plugs 20 or in the sixth plug 60.

In addition, as shown in the box C of Fig. 9, the magnetic particles M can be diffused in the second plug 20 by simply moving the permanent magnet 410 away from the tube 200. Since the magnetic particles M have a hydrophilic surface, the magnetic particles M have difficulty entering the oils of the first plug 10 and the third plug 30 even when, for example, the magnetic force is weakened and diffused in the second plug 20. Thus, such an aspect may be employed.

Specifically, when the magnetic particles M are moved from the container 120, pass through the first plug 10, and reach the second plug 20 using the permanent magnet 410, the permanent magnet 410 is moved away from the tube 200 to diffuse the magnetic particles M in the second plug 20. The magnetic particles M can be moved again, pass through the third plug 30, and be introduced to the fourth plug 40 using the magnetic force of the permanent magnet 410.

The aspect in which the magnetic particles M are vibrated, or repeatedly diffused and aggregated by changing the magnetic force applied from the outside may also be applied when the magnetic particles M exist in the adsorbent liquid in the container 120 or when the magnetic particles M exist in the fourth plug 40 (eluate).

### 3.8.2. Modification of Process of Eluting Nucleic Acid

In the above-described clause "3.5. Process of Eluting Nucleic Acid", the fourth plug 40 may be heated. Examples of the method of heating the fourth plug 40 include a method of bringing a heating medium such as a heating block into contact with a position corresponding to the fourth plug 40 of the tube 200, a method using a heat source such as a heater, and an electromagnetic heating method.

When the fourth plug 40 is heated, a plug other than the fourth plug 40 may be heated. However, in a state in which the magnetic particles M adsorbing the nucleic acid exist in the washing liquid plug, the plug is preferably not heated. The temperature which is reached when the fourth plug 40 is heated is preferably 35°C to 85°C, more preferably 40°C to 80°C, and even more preferably 45°C to 75°C from the viewpoint of elution efficiency and from the viewpoint of suppression of, when the eluate contains an enzyme for PCR, deactivation of the enzyme.

In the process of eluting the nucleic acid, when the fourth plug 40 is heated, the nucleic acid adsorbed to the magnetic particles M can be more efficiently eluted to the eluate. Even when the first or second washing liquid has a composition which is the same as or similar to the composition of the eluate, the nucleic acid remaining on and adsorbed to the magnetic particles M without being eluted to the washing liquid can be eluted to the eluate. That is, even after the magnetic particles M adsorbing the nucleic acid are washed with the first or second washing liquid, the nucleic acid can be further eluted to the eluate. Accordingly, sufficient washing and elution to the eluate at a sufficient concentration can be balanced even when the composition of the washing liquid and the composition of the eluate are the same as or similar to each other.

### 3.8.3. Modification of Process of Discharging Fourth Plug from Tube

When the above-described "3.7. Process of Discharging Fourth Plug from Tube" is employed, the magnetic particles M from which the adsorbed nucleic acid has been eluted to the eluate in the related process may exist in the fourth plug 40, but the magnetic particles M may be moved to any one of the first plug 10, the second plug 20, and the third plug 30 or to the container 120 by applying a magnetic force. Thus, the fourth plug 40 can be discharged from the tube 200 in a state in which the eluate does not contain the magnetic particles M. When a destination of the magnetic particles M is the second plug 20 or the container 120, the magnetic particles M have difficulty entering the oil of the third plug 30 even when the magnetic force is removed, and thus the fourth plug 40 can be more easily discharged from the tube 200.

### 4. Nucleic Acid Extraction Apparatus

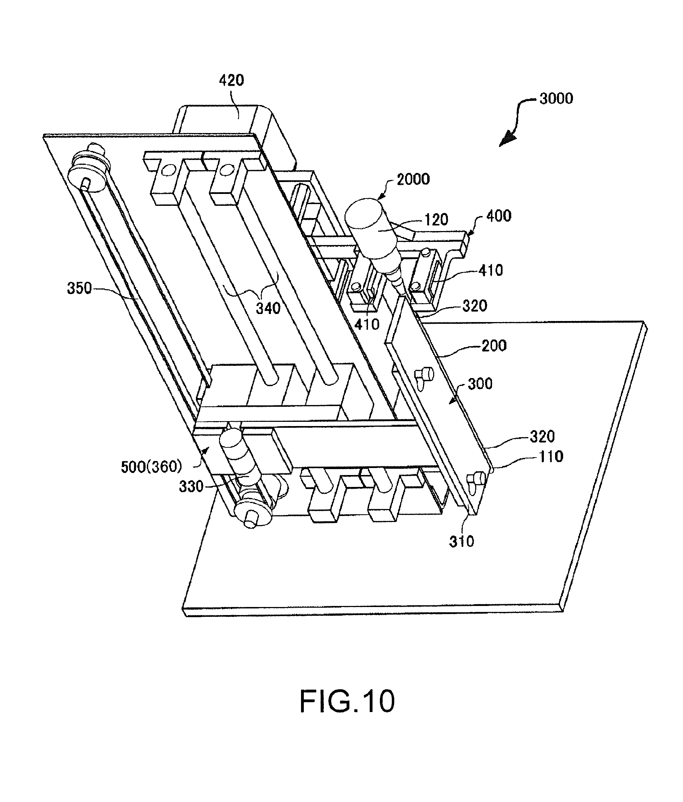

A nucleic acid extraction apparatus according to this embodiment can be appropriately applied to the nucleic acid extraction device, the nucleic acid extraction kit, and the nucleic acid extraction method which have been described above. Hereinafter, a nucleic acid extraction apparatus 3000 which extracts a nucleic acid with the nucleic acid extraction kit 2000 mounted thereon will be described as an embodiment. Fig. 10 is a perspective view schematically illustrating the nucleic acid extraction apparatus 3000 of this embodiment.

The nucleic acid extraction apparatus 3000 of this embodiment includes: a mounting portion 300 on which a tube having a longitudinal direction in which a first plug 10 formed of an oil, a second plug 20 formed of a first washing liquid which does not mix with an oil, a third plug 30 formed of an oil, a fourth plug 40 formed of an eluate which does not mix with an oil, and a fifth plug 50 formed of an oil are arranged in this order is mounted; a magnetic force application portion 400 which applies, when a tube 200 is mounted on the mounting portion 300, a magnetic force from a side surface of the tube 200; and a moving mechanism 500 which changes the relative arrangement between the mounting portion 300 and the magnetic force application portion 400 in the longitudinal direction of the tube 200.

The tube 200 mounted on the mounting portion 300 of the nucleic acid extraction apparatus 3000 is the above-described tube 200. The nucleic acid extraction apparatus 3000 has the mounting portion 300 on which the tube 200 is mounted. Although an example has been shown in which the plugs ranging from the first plug 10 to the fifth plug 50 are arranged in the tube 200, the above-described sixth plug 60 and seventh plug 70 may also be arranged.

The mounting portion 300 is a portion in which the tube 200 is mounted. Together with the tube 200, a container 120 connected to the tube 200 may also be mounted on the mounting portion 300. As the mounting portion 300, a mechanism or the like for configuration or mounting can be appropriately designed within a range in which the magnetic force application portion 400 can apply a magnetic force to the tube 200, and if necessary, to the container 120. The mounting portion 300 may be configured so that when the tube 200 is flexibly bent, the tube 200 can be mounted by being stretched into a linear shape. In addition, in the example illustrated in Fig. 10, the mounting portion 300 has a doubling plate 310 disposed along the tube 200. The doubling plate 310 is not an essential configuration. However, in some cases, vibration of the tube 200 can be suppressed when the doubling plate 310 is installed. In the example illustrated in Fig. 10, the mounting portion 300 has clip mechanisms 320, and thus the tube 200 is fixed at two places.

The mounting portion 300 is configured so that the positional relation with the magnetic force application portion 400 is relatively changed in the longitudinal direction of the tube 200. Accordingly, when a design in which the mounting portion 300 is relatively moved with respect to the magnetic force application portion 400 with no movement of the magnetic force application portion 400 is provided, a moving mechanism 360 which moves the mounting portion 300 is included as the moving mechanism 500 as illustrated in Fig. 10. In some cases, the moving mechanism 360 is not required for the mounting portion 300 when the magnetic force application portion 400 includes a moving mechanism. In the example illustrated in Fig. 10, the mounting portion 300 is configured to include a hinge 330, guide rails 340, a drive belt 350, and a motor (not illustrated).

In the example of the nucleic acid extraction apparatus 3000, one mounting portion 300 is provided, but more than one mounting portion 300 may be provided. In that case, more than one magnetic force application portion 400 can be provided, and the plural mounting portions 300 may be provided separately or in conjunction with each other.

The magnetic force application portion 400 is a configuration for applying a magnetic force to the tube 200, and if necessary, to the container 120 when the tube 200 is mounted on the mounting portion 300. The magnetic force application portion 400 is configured to include, for example, a permanent magnet, an electromagnet, or a combination thereof. The magnetic force application portion 400 is provided with at least one magnet. However, more than one magnet may be provided. It is preferable that an electromagnet not be used, but a permanent magnet be used in the magnetic force application portion 400 since generation of heat and the like are difficult to occur. As the permanent magnet, for example, a nickel-based, iron-based, cobalt-based, samarium-based, or neodymium-based permanent magnet can be used.

The magnetic force application portion 400 functions to apply a magnetic force to magnetic particles M which exist in the container 120 and in the tube 200. The magnetic particles M can be moved in the container 120 and in the tube 200 by changing the relative positional relation between the mounting portion 300 and the magnetic force application portion 400.

In the example illustrated in Fig. 10, the magnetic force application portion 400 has a pair of permanent magnets 410 provided opposed to each other with the container 120 and the tube 200 interposed therebetween. The pair of permanent magnets 410 is separated from each other at an interval larger than an external diameter of the tube 200. The direction of the polarity of the permanent magnet 410 is not particularly limited. The magnetic force application portion 400 is configured so that the positional relation with the mounting portion 300 is relatively changed in the longitudinal direction of the tube 200. Accordingly, when a design in which the magnetic force application portion 400 is relatively moved with respect to the mounting portion 300 with no movement of the mounting portion 300 is provided, a moving mechanism which moves the magnetic force application portion 400 is included as the moving mechanism 500.

In addition, in the example illustrated in Fig. 10, the magnetic force application portion 400 is disposed so that when one of the pair of permanent magnets 410 is moved closer to the tube 200, the other one is separated from the tube 200. Vibration can be applied using a motor 420 so that the pair of permanent magnets 410 is moved closer to or separated from the tube 200. The magnetic particles M can be moved to reciprocate in a direction crossing the longitudinal direction of the tube 200 in the tube 200 by driving the motor 420.

If necessary, the motor 420 can also be driven when a magnetic force is applied to any of the container 120 and the tube 200. Efficiency of washing the magnetic particles M in the tube 200 or elution efficiency can be increased when the motor 420 is driven at the time when the permanent magnet 410 is positioned at the position of the second plug 20 or the position of the fourth plug 40 of the tube 200.

According to the nucleic acid extraction apparatus 3000 of this embodiment, preprocessing for PCR can be automated, and the time and effort required for the preprocessing can be significantly reduced. In addition, according to the nucleic acid extraction apparatus 3000 of this embodiment, since the magnetic force application portion 400 can be vibrated, washing (purification) of magnetic particles M adsorbing a nucleic acid can be more efficiently performed, and thus the accuracy of PCR can be further increased.

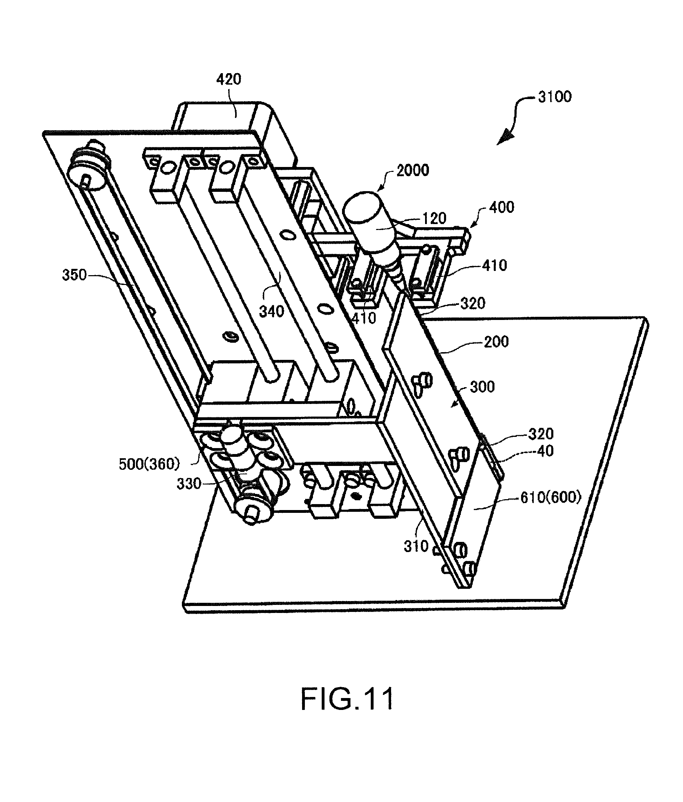

Fig. 11 is a perspective view schematically illustrating a nucleic acid extraction apparatus 3100 according to a modification example of the nucleic acid extraction apparatus. The nucleic acid extraction apparatus 3100 is the same as the above-described nucleic acid extraction apparatus 3000, except that a heating portion 600 is provided. Members having common actions and functions will be denoted by the same reference numerals, and description thereof will be omitted.

The heating portion 600 is configured to heat a part of the tube 200 when the tube 200 is mounted on the mounting portion 300. Examples of the heating portion 600 include a heat source, a heating block, a heater, and a coil for electromagnetic heating. The heating portion 600 is shaped to allow insertion of the tube 200 therein or to be brought into contact with the side surface of the tube 200, and is arbitrarily shaped as long as the liquid in the tube 200 can be heated.

The part heated by the heating portion 600 in the tube 200 includes a part where the fourth plug 40 exists in the longitudinal direction of the tube 200. The heating portion 600 may heat another part of the tube 200, but preferably does not heat a part where the second plug 20 exists in the longitudinal direction of the tube 200.

The nucleic acid extraction device 3100 illustrated in Fig. 11 is provided with, as the heating portion 600, a heater 610 which is provided in parallel to the doubling plate 310 to heat a position including the fourth plug 40 of the tube 200. The heater 610 is shaped to be brought into contact with approximately half of the outer periphery of the tube 200.

The nucleic acid extraction device 3100 can elute a sufficient amount of a nucleic acid to the eluate of the fourth plug 40 even when the amount of the nucleic acid adsorbed to magnetic particles M is reduced by washing with at least one of the first washing liquid of the second plug 20 and the second washing liquid of the sixth plug 60. Accordingly, the washing effect can be increased, and a sufficient concentration of a nucleic acid for PCR can be eluted to the eluate.

### 5. Batching

When using the adsorbent liquid, the first washing liquid, the eluate, and the optional second washing liquid which have been described above, it is possible to efficiently perform a reverse transcription reaction or a nucleic acid amplification reaction in batching which does not use the above-described nucleic acid extraction device or nucleic acid extraction apparatus.

For example, a nucleic acid-containing sample is put into one tube and mixed with a chaotropic substance-containing adsorbent liquid and fine particles to adsorb the nucleic acid to the fine particles. Thereafter, the fine particles are precipitated by centrifugation and a supernatant is removed. A first washing liquid is added to the fine particles adsorbing the nucleic acid which remain in the tube to suspend the fine particles, and centrifugation is performed again to remove a supernatant. Thus, the fine particles are washed. The fine particles may be washed with a second washing liquid before the washing with the first washing liquid.

Next, an eluate is added to the fine particles in the tube to elute the nucleic acid adsorbed to the fine particles to the eluate, and the eluate obtained in this manner is used. When the nucleic acid is mRNA, it is reverse-transcribed to synthesize cDNA and the synthesized cDNA is amplified, and when the nucleic acid is genomic DNA or cDNA, the DNA can be amplified as is.

### 6. Examples

### 6.1. Experimental Examples 1 and 2

In this example, experimental examples using batching will be described.

### Isolation of RNA

First, 700 µL of an adsorbent liquid was put into a polyethylene container having a capacity of 1.5 mL, and 140 µL of a solution containing an influenza A positive sample (nasal cavity wiping liquid) and 25 µL of fine magnetic particles (magnetic beads having a silica surface with a diameter of 1 µm to 3 µm, and adsorbent liquid at a concentration of 400 mg/mL) were put thereinto. Stirring was sufficiently performed for 5 minutes using a vortex mixer. The magnetic beads were attracted by a magnet to remove the adsorbent liquid. Next, a washing liquid A was put and stirring was performed for 10 seconds using the vortex mixer. The fine magnetic particles were attracted by a magnet to remove the washing liquid A. After the washing with the washing liquid A was performed two times, washing with a washing liquid B was performed two times similarly. The washing liquid was removed, and then 27.5 µL of water as an eluate was added to the magnetic beads and stirring was lightly performed for 5 seconds using the vortex mixer. Then, heating was performed for 3 minutes at 65°C. Finally, the magnetic beads were attracted by the magnet to recover the eluate, and this eluate was a RNA solution.
Adsorbent Liquid

**Experimental Example 1:**

| | |
|---|---|
| Guanidine Thiocyanate | 60 wt% |
| Triton X-100 | 2.0 vol.% |
| Tris (pH 7.2) | 0.6 wt% |

**Experimental Example 2:**

| | |
|---|---|
| Guanidine Thiocyanate | 30 wt% |
| Ethanol | 45% vol.% |
| Tris (pH 7.3) | 0.3 wt% |

Washing liquid A

**Experimental Example 1:**

| | |
|---|---|
| Guanidine Thiocyanate | 70 wt% |
| Triton X-100 | 0.7 vol.% |
| Tris (pH 7.2) | 2.6 wt% |

**Experimental Example 2:**

| | |
|---|---|
| Guanidine Thiocyanate | 30 wt% |
| Ethanol | 57 wt% |

Washing liquid B

**Experimental Example 1:**

| | |
|---|---|
| Tris (pH 7.5) | 3 wt% |

**Experimental Example 2:**

| | |
|---|---|
| Tris (pH 7.0) | 0.1 wt% |
| Ethanol | 70 vol.% |

### RT-PCR

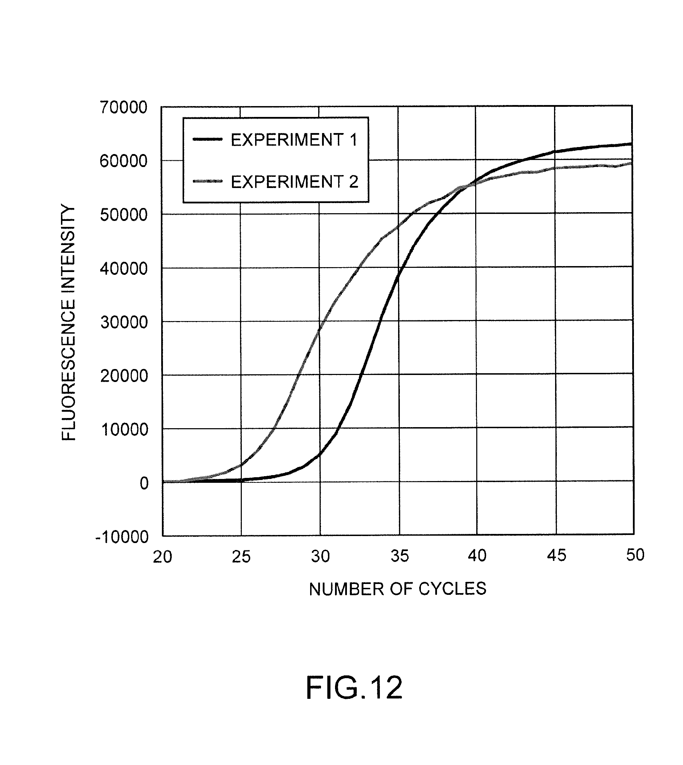

Next, RT-PCR was performed using the prepared RNA solution. The RT-PCR was carried out according to CDC protocol of real-time RTPCR for swine influenza A (H1N1) . Specifically, 8 µL of a solution containing: 0.8 µM of a forward primer; 0.8 µM of a reverse primer; 0.2 µM of a probe; 1x SuperScript III RT/Platimun Taq Mix; and 1x PCR Master Mix was prepared, and 2 µL of an eluate was added thereto. After stirring, the RT-PCR was performed under the following conditions. The results thereof are illustrated in Fig. 12.

| | |
|---|---|
| Reverse Transcription | 50°C, 30 minutes |
| Taq Activation | 95°C, 2 minutes |
| PCR | 95°C, 15 seconds-55°C, 30 seconds; 50 cycles |

When comparing the results of Experimental Example 1 and Experimental Example 2, a rise in fluorescence intensity of the RT-PCR was shown earlier in Experimental Example 2. This fact indicates that when an alcohol is added to the adsorbent liquid, the washing liquid A, and the washing liquid B, RNA recovery efficiency is improved. Similarly, an improvement in the recovery efficiency was also shown when methanol or acetonitrile was used in place of the ethanol.

### 6.2. Experimental Examples 3 and 4

### Isolation of RNA

Next, extraction of RNA was performed using a nucleic acid extraction device having a sixth plug 60 and a seventh plug 70 in the tube 200 in the nucleic acid extraction kit 2000 illustrated in Figs. 7A and 7B. In Experimental Example 3, the same reagent as that of Experimental Example 1 of the clause 6.1 was used, and in Experimental Example 4, the same reagent as that of Experimental Example 2 of the clause 6.1 was used. However, to an eluate, a reagent for RT-PCR was added in advance, and 0.2 wt% of bovine serum albumin was further added thereto. The reagent amounts of the plugs are as follows.

| | |
|---|---|
| Sixth Plug: | 28 µL of Washing Liquid A |
| Second Plug: | 22 µL of Washing Liquid B |
| Fourth Plug: | 1.6 µL of Eluate |
| First, Third, Fifth, and Seventh Plugs: | 10 µL of Oil |

The method of operating the nucleic acid extraction device will be described below. First, 700 µL of an adsorbent liquid was put into a polyethylene container 120 having a capacity of 3 mL. 140 µL of a solution containing an influenza A positive sample (nasal cavity wiping liquid) and 1 µL of fine magnetic particles similar to those of the clause 6.1 were put thereinto. The container was covered with a lid and shaken for stirring for 30 seconds by hand. The lid of the container was removed and the container was connected to a side of the first plug of the tube. The magnetic beads in the container were introduced into the tube by moving a permanent magnet by hand. The magnetic beads was vibrated and moved up to the sixth plug as illustrated in Fig. 9. A time during which the magnetic beads existed in each plug in the tube was roughly as follows. In the fifth plug, the operation of vibrating the magnetic beads was not performed.

| | |
|---|---|
| First, Seventh, Third Plugs: | 3 seconds |
| Sixth Plug: | 20 seconds |
| Second Plug: | 30 seconds |
| Fourth Plug: | 20 seconds |

Next, the fourth plug 40 of the tube was heated at 50°C and vibrated for 30 seconds to extract RNA to the eluate from the magnetic beads. Thereafter, the magnetic beads were moved and retreated up to the sixth plug using the permanent magnet. The cock on the side of the fifth plug of the tube was removed and the container was deformed by hand to discharge the fifth plug to the container and to discharge the fourth plug to the rotation-type PCR apparatus disclosed in JP-A-2012-115208, and thus droplets were formed in the oil.

### RT-PCR

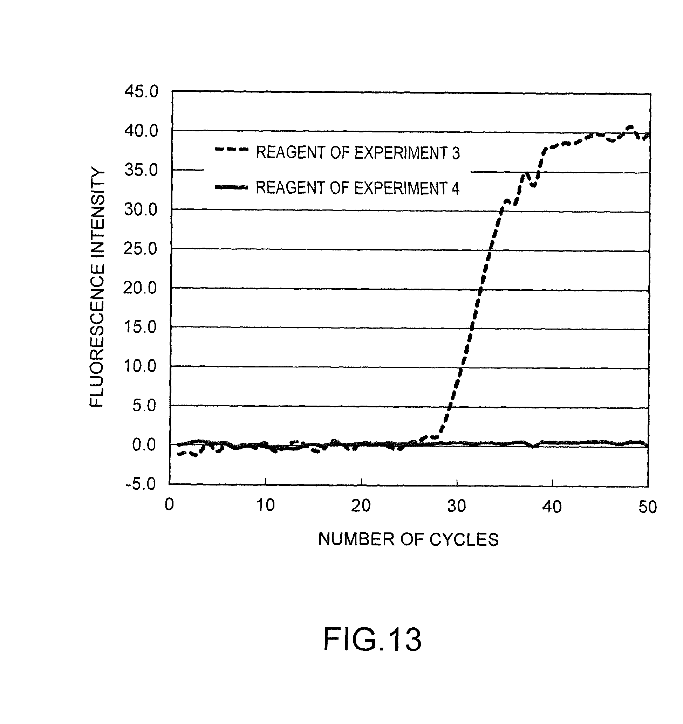

Real-time PCR was performed under the following conditions using the obtained RNA solution. The results thereof are illustrated in Fig. 13.

| | |
|---|---|
| Reverse Transcription | 50°C, 60 seconds |
| Taq Activation | 100°C, 10 seconds |
| PCR | 100°C, 10 seconds-57°C, 30 seconds; 50 cycles |

In Experimental Example 3, DNA amplification was normally observed, but in Experimental Example 4, it was not possible to detect DNA amplification. The reason for this was thought to be that in Experimental Example 4, the adsorbent liquid, the washing liquid A, and the washing liquid B contained the ethanol and carry-over thereof inhibited the PCR.

### 6.3. Example

### Isolation of RNA

Isolation of RNA was performed using the following solutions with similar apparatus and procedures to those of the clause 6.2.

**Adsorbent Liquid**

| | |
|---|---|
| Guanidine Thiocyanate | 30 wt% |
| Ethanol | 45 vol.% |
| Tris (pH 7.3) | 0.3 wt% |

**Washing Liquid B**

| | |
|---|---|
| Tris (pH 7.0) | 0.1 wt% |
| Ethanol | 70 vol.% |

**Washing Liquid C**

| | |
|---|---|
| Citric Acid (pH 4.0) | 0.1 wt% |

In this example, a plug corresponding to the washing liquid A of the clause 6.2. was omitted, instead, a plug formed of a washing liquid C was provided on the downstream side of a plug formed of a washing liquid B. RT-PCR was performed according to similar procedures to those of Experiment 2 with the presence or absence of the washing liquid A, and as a result, no change in RNA recovery efficiency was confirmed. The washing liquid A may be used when a sample required to be more frequently washed, such as a sample with a large amount of foreign substances, was used.

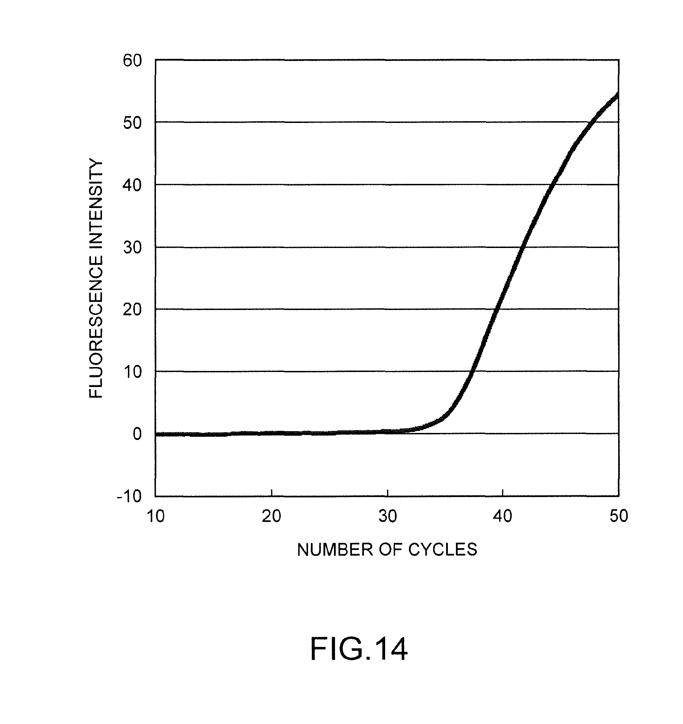

As illustrated in Fig. 14, DNA amplification was observed, though it was not recognized in Experimental Example 4. By performing the washing process with an acidic solution, it was possible to avoid inhibition of the reverse transcription reaction and/or the nucleic acid amplification reaction by carry-over of the ethanol. In this example, the occurrence of the DNA amplification is confirmed even when the washing process with the washing liquid B is omitted.

In addition, it is confirmed that even when additional washing with the washing liquid C is performed in the batching described in the clause 6.1, the extraction efficiency, that is, the rapid rise of the amplification curve in the real-time PCR is the same as in Example 2. That is, using an acidic washing liquid, it was possible to achieve two effects, i.e., an improvement in efficiency of the extraction using the reagent containing the ethanol and prevention of the bringing of the ethanol into the eluate.

The invention is not limited to the above-described embodiments, and various modifications can be made. For example, the invention includes configurations substantially the same as those described in the embodiments (for example, configurations having substantially the same functions, methods, and results, and configurations having substantially the same objects and effects). The invention also includes configurations in which non-essential parts of the configurations described in the embodiments are replaced with others. The invention also includes configurations that achieve the same advantages or achieve the same objects as those of the configurations described in the embodiments. The invention also includes configurations in which known techniques are added to the configurations described in the embodiments.

## Claims

1. A nucleic acid amplification method comprising:
adsorbing a nucleic acid to fine particles by mixing a chaotropic substance-containing adsorbent liquid and the fine particles with a nucleic acid-containing sample;
washing the fine particles adsorbing the nucleic acid with a first washing liquid;
eluting the nucleic acid adsorbed to the fine particles in an eluate; and
performing a nucleic acid amplification reaction on the nucleic acid in the eluate,
wherein the adsorbent liquid contains an alcohol, and the first washing liquid is acidic.

2. The nucleic acid amplification method according to claim 1,
wherein the alcohol in the adsorbent liquid is methanol or ethanol.

3. The nucleic acid amplification method according to claim 1,
wherein the adsorbent liquid has an alcohol concentration of 40% to 50% by volume.

4. A nucleic acid amplification method according to claim 1, further comprising after the adsorbing step but before the washing step, a further step of
washing the fine particles adsorbing the nucleic acid with a second washing liquid which contains an alcohol or acetonitrile.

5. The nucleic acid amplification method according to claim 4,
wherein the alcohol in the second washing liquid is methanol or ethanol.

6. The nucleic acid amplification method according to claim 4,
wherein a concentration of the alcohol in the second washing liquid is 70% to 100% by volume.

7. The nucleic acid amplification method according to claim 1,
wherein the nucleic acid is a ribonucleic acid (RNA), wherein reverse-transcribing the ribonucleic acid eluted in the eluate to synthesize cDNA is further included, and
wherein the nucleic acid which is amplified in the amplifying the nucleic acid is the synthesized cDNA.

8. The nucleic acid amplification method according to claim 1,
wherein the reaction liquid in the amplifying the nucleic acid contains 50% by volume or more of the eluate.

9. The nucleic acid amplification method according to claim 8,
wherein the reaction liquid in the amplifying the nucleic acid is the eluate.

10. A nucleic acid extraction device comprising:
a tube having a longitudinal direction in which a first plug formed of an oil, a second plug formed of a first washing liquid which is phase-separated from an oil and is provided to wash fine particles to which a nucleic acid is bonded, a third plug formed of an oil, a fourth plug formed of an eluate which is phase-separated from an oil and is provided to elute the nucleic acid from the fine particles to which the nucleic acid is bonded, and a fifth plug formed of an oil are provided in order; and either
(i) a container which is capable of being connected to and allowed to communicate with a side of the tube where the first plug is disposed, and which contains an adsorbent liquid for adsorbing the nucleic acid to the fine particles, or
(ii) a liquid reservoir which communicates with a side of the tube where the first plug is disposed, and which contains an adsorbent liquid for adsorbing the nucleic acid to the fine particles,
wherein the adsorbent liquid contains an alcohol, and the first washing liquid is acidic.

11. The nucleic acid extraction device according to claim 10,
wherein the fourth plug has a volume of 0.8 µL to 5 µL.

12. The nucleic acid extraction device according to claim 10,
wherein the alcohol in the adsorbent liquid is methanol or ethanol.

13. The nucleic acid extraction device according to claim 10,
wherein the adsorbent liquid has an alcohol concentration of 40% to 50% by volume.

14. A nucleic acid extraction device according to claim 10 or 12, further comprising, after the first plug but before the second plug, a sixth plug formed of a second washing liquid which is phase-separated from an oil and is provided to wash fine particles to which a nucleic acid is bonded and a seventh plug formed of an oil,
wherein the second washing liquid contains an alcohol or acetonitrile.

15. The nucleic acid extraction device according to claim 14,
wherein the second washing liquid has an alcohol concentration of 70% to 100% by volume.

16. A nucleic acid amplification reaction cartridge comprising:
the nucleic acid extraction device according to claim 10; and
a nucleic acid amplification reaction container which communicates with a side of the tube where the fifth plug is disposed, and which contains an oil.

17. The nucleic acid amplification reaction cartridge according to claim 16,
wherein a reaction liquid of a nucleic acid amplification reaction contains 50% by volume or more of an eluate in which the nucleic acid is eluted.

18. The nucleic acid amplification reaction cartridge according to claim 17,
wherein the reaction liquid of the nucleic acid amplification reaction is the eluate.

19. The nucleic acid amplification reaction cartridge according to claim 16, further comprising:
a tank which communicates with a side of the tube where the first plug is disposed, and which introduces the fine particles to the tube.

20. A nucleic acid amplification reaction kit comprising:
the nucleic acid extraction device according to claim 14; and
a tank which introduces the fine particles to the tube.
